(19) Europäisches Patentamt
European Patent Office
Office européen des brevets

(11) EP 2 381 259 B1

(12) # EUROPEAN PATENT SPECIFICATION

(45) Date of publication and mention of the grant of the patent:
**21.09.2016 Bulletin 2016/38**

(21) Application number: **09834674.5**

(22) Date of filing: **26.11.2009**

(51) Int Cl.:
***G01N 33/68*** *(2006.01)* ***C07K 5/02*** *(2006.01)*
***G01N 27/62*** *(2006.01)* ***G01N 33/576*** *(2006.01)*
***G01N 33/497*** *(2006.01)* ***G01N 33/487*** *(2006.01)*

(86) International application number:
**PCT/JP2009/069950**

(87) International publication number:
**WO 2010/073870 (01.07.2010 Gazette 2010/26)**

(54) **METHOD FOR MEASURING LIVER DISEASE MARKERS AND TEST METHOD FOR PHARMACEUTICAL PREPARATION**

VERFAHREN ZUR MESSUNG VON LEBERKRANKHEITSMARKER SOWIE TESTVERFAHREN FÜR DIE PHARMAZEUTISCHE HERSTELLUNG

PROCÉDÉ POUR MESURER MARQUEUR DE MALADIE DU FOIE ET PROCÉDÉ D'ANALYSE POUR UNE PRÉPARATION PHARMACEUTIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.12.2008 JP 2008328133**

(43) Date of publication of application:
**26.10.2011 Bulletin 2011/43**

(73) Proprietor: **Keio University**
**Tokyo 108-8345 (JP)**

(72) Inventors:
- **SOGA, Tomoyoshi**
**Tsuruoka-shi**
**Yamagata 997-0017 (JP)**
- **SUGIMOTO, Masahiro**
**Tsuruoka-shi**
**Yamagata 997-0017 (JP)**
- **SUEMATSU, Makoto**
**Tokyo 160-8582 (JP)**
- **HONMA, Masashi**
**Tokyo 113-8654 (JP)**
- **YAMAMOTO, Takehito**
**Tokyo 113-8654 (JP)**
- **SUZUKI, Hiroshi**
**Tokyo 113-8655 (JP)**

(74) Representative: **Grünecker Patent- und Rechtsanwälte**
**PartG mbB**
**Leopoldstraße 4**
**80802 München (DE)**

(56) References cited:
**EP-A1- 1 626 280** **EP-A1- 1 975 621**
**WO-A1-99/33799** **WO-A1-2007/083632**
**WO-A1-2010/017178** **WO-A2-2007/057548**
**WO-A2-2009/059150** **JP-A- H09 506 336**
**JP-A- 2000 083 698** **US-A1- 2008 311 593**

- **NAGY K ET AL: "Effect of glutaurine on liver tumour development and acute leukaemia induced by MC29 virus in turkey poults", ACTA MICROBIOLOGICA ACADEMIAE SCIENTIARUM HUNGARICA, SCIENCES, BUDAPEST, vol. 30, no. 1, 1 January 1983 (1983-01-01), pages 37-42, XP008150739, ISSN: 0231-4622**
- **BOUDONCK K J ET AL: "Discovery of metabolomics biomarkers for early detection of nephrotoxicity", TOXICOLOGIC PATHOLOGY, SAGE SCIENCE PRESS (US), US, vol. 37, no. 3, 1 January 2009 (2009-01-01), pages 280-292, XP008111286, ISSN: 1533-1601, DOI: 10.1177/0192623309332992**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ’Soyaku Vision Symposium Koen Yoshishu, 21 January 2008 (21.01.2008)’, vol. 9TH, article TOMOYOSHI SOGA: ’Saishin no Metabolome Kaiseki ni yoru Biomarker no Tansakuho’, pages 26 - 27
- YOSHISHIGE URATA ET AL.: ’Sanka Stress no Kiso 18 Redox Seigyo y-glutamylcystein Synthetase Idenshi no Hatsugenchosetsu to Igi’ SHUKAN IGAKU NO AYUMI vol. 10 GATSU, 2006, pages 74 - 78
- JANDKE J A ET AL.: ’Dipeptide analysis in human urine’ J CHROMATOGR vol. 382, 1986, pages 39 - 45

## Description

TECHNICAL FIELD

The present invention relates to a liver disease marker, a method and an apparatus for measuring the same, and a method for assaying a pharmaceutical preparation. In particular, the present invention relates to a liver disease marker that allows for screening to distinguish patients with various liver diseases from normal persons, a method and an apparatus for measuring the same, and a method for assaying a pharmaceutical preparation by using the liver disease marker.

BACKGROUND ART

There are various types of liver diseases, such as drug-induced hepatitis, hepatitis B, hepatitis C, hepatic cirrhosis, and liver cancer. There are also asymptomatic carriers of a B-type virus or a C-type virus. In particular, 70 percent of hepatitis C virus (HCV)-infected individuals experience gradual loss of normal stem cells, fibrosis of the liver, progression to hepatic cirrhosis, and furthermore development of liver cancer, due to chronic liver inflammation (chronic hepatitis). It is reported that 10 to 15 % of chronic hepatitis C patients and 80 % of hepatic cirrhosis patients develop liver cancer. Although the state of chronic hepatitis is not life-threatening, life is threatened when liver cancer develops or hepatic cirrhosis progresses to cause hepatic failure. Therefore, it is necessary to diagnose hepatitis C at an early stage and disinfect the virus.

Hepatitis C progresses from hepatic cirrhosis to liver cancer with no symptoms, and liver function deteriorates extremely resulting in various disorders such as malaise, jaundice, and disturbed consciousness. However, at this stage, there is currently no effective therapy. Therefore, it is necessary to detect progression of the symptoms as early as possible before liver function deteriorates and to apply a treatment such as interferon administration. However, there is currently no established method for identifying various liver injuries precisely and rapidly.

Generally, when a liver disease is suspected, liver function markers such as AST, ALT, γ-GPT, alkaline phosphatase (AL-P), choline esterase (ChE), and bilirubin in blood are measured in conjunction with a medical examination by interview, an inspection, and a palpation. When an abnormality was found in these biochemical values, a hepatitis B virus test and a hepatitis C virus test, and imaging tests such as an ultrasound examination, an X-ray examination, and a CT examination are performed. For determination of cancer, proteinous tumor markers such AFP, PIVKA-II, and CEA in blood are measured. Furthermore, when an accurate determination is required, laparoscopy, liver biopsy, and the like (about one week of hospital stay is required) are performed (Non Patent Literature 1).

Thus, identification of liver diseases requires many examinations and it takes many days for the disease to be determined. Laparoscopy, liver biopsy, and the like also endanger patients and cause them physical pain. Since laparoscopy, liver biopsy, and the like put a heavy burden on patients, they may not be performed frequently to check the patients' pathological conditions. Furthermore, in the case of conventional methods, many of the examinations or determinations may be performed by only experts, and therefore burden is imposed on insufficient health care practitioners. Therefore, a method for determining a liver disease rapidly, precisely, and conveniently without putting a burden on patients is highly desirable.

Many liver injuries such as hepatitis, hepatic cirrhosis, and liver cancer are known to be caused by generation of active oxygen (oxidative stress) and disruption of the protection system of a living organism to remove it (Non Patent Literature 2). One of the major protection systems of a living organism against oxidative stress such as active oxygen is a glutathione system. Reduced glutathione (GSH: referred to as glutathione hereinbelow) is an antioxidant that exists in the highest concentration in a tissue. Glutathione conjugates to active oxygen, electrophiles, and the like, and reduces these substances, thereby suppressing oxidative stress.

However, when the glutathione is decreased, a tissue, a cell, and the like are exposed to oxidative stress, and various pathological conditions are caused (Non Patent Literature 3). In fact, it is reported that, in liver injuries, oxidative stress is increased by infection with a hepatitis B virus or a hepatitis C virus, and glutathione is decreased, and that, in patients and mice with hepatitis C, hepatic cirrhosis, or liver cancer, glutathione is decreased (Non Patent Literatures 2 and 4).

Drug-induced hepatitis, which is induced by taking a drug, is also caused by oxidative stress. Acetaminophen (APAP), which is an antipyretic analgesic, is metabolized in the liver to generate a highly toxic electrophile, N-acetylbenzoquinoneimine (NAQPI). This NAQPI is conjugated to by glutathione (GSH), which exists in a high concentration in the liver, and is detoxified and excreted. However, when the electrophiles exist in large quantities, the glutathione is depleted, and the electrophiles accumulate in cells (oxidative stress) and react with a biopolymer. It is known that cellular functions are consequently disturbed, thereby causing pathological conditions such as drug-induced hepatitis.

Previously, the present inventors have found that glutathione was decreased in order to detoxify electrophiles, NAQPIs, generated by metabolism of APAP, and ophthalmic acid was increased rapidly in inverse proportion to the glutathione level, when large quantities of APAP were administered to a mouse (see Fig. 1(B)). The present inventors also have found that an increase of ophthalmic acid in the liver and blood indicates depletion of glutathione in the liver caused by the electrophiles (Patent Literature 1, Non Patent Literature 5).

The mechanism is as follows. As shown in Fig. 1, glutathione (γ-Glu-Cys-Gly) and ophthalmic acid (γ-Glu-2AB-Gly) are tripeptides biosynthesized by the same two enzymes, a γ-glutamylcysteine synthetase and a glutathione synthetase. They are different in their substrates (starting materials), which are cysteine (Cys) and 2-aminobutyric acid (2AB). In the normal reduction state shown in Fig. 1(A), glutathione exists in large quantities in the liver and the first enzyme, γ-glutamylcysteine synthetase is under feedback (FB) inhibition. Therefore, little ophthalmic acid is biosynthesized. However, when electrophiles, active oxygen species, and the like exist in such a case as an oxidation state shown in Fig. 1(B), glutathione is consumed for detoxication. Feedback inhibition is canceled due to the decrease of glutathione, γ-glutamylcysteine synthetase is activated, and glutathione and ophthalmic acid are biosynthesized. Ophthalmic acid accumulates in the liver and also is excreted into the blood. As described above, since ophthalmic acid in the liver, blood, and the like increases under an oxidation state caused by an electrophile and the like, ophthalmic acid serves as a biomarker of oxidative stress.

Further, nonalcoholic fatty liver diseases (NAFLD) occur when visceral fat increases because of obesity. With respect to nonalcoholic fatty liver diseases (NAFLDs), it is also reported that serum thioredoxin (TRX), a marker of oxidative stress, is useful for distinguishing between nonalcoholic steatohepatitis (NASH) that progresses to hepatic cirrhosis and further to liver cancer and simple steatosis (SS) that has a favorable course, (Non Patent Literature 6). [0012]

On the other hand, there is a comprehensive method for measuring metabolites in a cell, which is based on a method of measuring metabolites in a sample by a capillary electrophoresis-mass spectroscope (CE-MS) (for example, see Non Patent Literatures 7 to 9). This comprehensive method includes determining a low molecular weight compound (metabolite) pattern and/or a peptide pattern of a liquid sample derived from a human body or an animal body qualitatively and/or quantitatively in order to monitor the condition of the human body or the animal body, wherein the metabolite and the peptide in the liquid sample are separated by capillary electrophoresis, subsequently directly ionized, and then detected on a mass spectrometer connected on-line through the interface. The reference value and the sample value that show the condition, and the deviation and correspondence derived from the values are automatically stored in a database in order to monitor the condition of the human body or the animal body over a prolonged period. When an anionic compound is separated and analyzed by combining capillary electrophoresis and mass spectrometry, a method for separating and analyzing an anionic compound including reversing electroosmotic flow by using a coated capillary whose inner surface is pre-coated cationically is known (for example, see Patent Literature 2).

CITATION LIST

PATENT LITERATURE

Patent Literature 1: Japanese Patent Application Laid-Open No. 2007-192746
Patent Literature 1: Japanese Patent No. 3341765 Non Patent Literature

Non-Patent Literature 1: Callewaert, N. et al. Nat. Med. 10, 429-434, 2004.
Non-Patent Literature 2: Loguercio, Carmela et al. Free Radic. Biol. Med. 34, 1-10, 2003.
Non-Patent Literature 3: Yadav, Dhiraj et al. Am. J. Gastroenterol. 97, 2634-2639, 2002.
Non-Patent Literature 4: Moriya, K. et al. Cancer Res. 61, 4365-4370, 2001.
Non-Patent Literature 5: Soga, T. et al. J. Biol. Chem. 281, 16768-16776, 2006.
Non-Patent Literature 6: Kyuichi Tanikawa eds., "Sanka Storesu to Kanshikkan Vol. 5 (in Japanese) (Oxidative Stress and Liver Diseases Vol. 5)" Medical Tribune, Inc., pages 3 - 37, 2009. 5. 7.
Non-Patent Literature 7: Soga, T. et al. J. Proteome Res.2. 488-494, 2003.
Non-Patent Literature 8: Soga, T. et al. J. Boil Chem. Vol. 281, No.24 (June 16, 2006), 16768-16776
Non-Patent Literature 9: Hirayama A. et al. Cancer. Res. 69: (II). (June 1, 2009) 4918-4925
Non-Patent Literature 10: Pignatelli, B. et al. Am. J. Gastroenterol. 96, 1758-1766, 2001.

SUMMARY OF INVENTION

TECHNICAL PROBLEM

However, it has been difficult so far to distinguish and identify drug-induced hepatitis, hepatitis B, hepatitis C, hepatic cirrhosis, liver cancer, asymptomatic carriers of a B-type virus or a C-type virus, and the like in one examination.
The present invention was achieved to solve the above-mentioned conventional problems. It is an object of the present invention to enable one to identify rapidly liver diseases such as drug-induced hepatitis, hepatitis B, hepatitis C, hepatic cirrhosis, liver cancer, asymptomatic carriers of a B-type virus or a C-type virus, a nonalcoholic fatty liver disease (NAFLD), nonalcoholic steatohepatitis (NASH), and simple steatosis (SS) by measuring a low molecular weight biomarker in blood.

MEANS FOR SOLVING THE PROBLEMS

Since many liver injuries such as hepatitis, hepatic cirrhosis, and liver cancer are closely related to oxidative stress as described above, the concentration of ophthalmic acid was expected to vary in the liver injuries. Thus, blood was collected from a normal person (i.e., a control: C) and patients with drug induced liver injury (DI), an asymptomatic hepatitis B carrier (AHB), an asymptomatic hepatitis C carrier (AHC), chronic hepatitis B (chronic hepatitis B carrier: CHB), chronic hepatitis C (chronic hepatitis C carrier: CHB), and liver cancer (hepatocellular carcinoma: HCC), and ophthalmic acid in the sera was measured. However, unlike mice, little ophthalmic acid was detected in the normal person, the drug-induced hepatitis patient, and the like. (While the concentration of ophthalmic acid in the murine serum was approximately 2 $\mu$M, its concentration in the human serum was approximately one twentieth the concentration in the murine serum and little ophthalmic acid was detected in the normal person, the drug-induced hepatitis patient, and the like).
However, the present inventors discovered substances that increased significantly in the sera of the hepatitis patients and identified the substances as $\gamma$-Glu-X peptides (notes: X represents an amino acid).
Furthermore, the present inventors successfully distinguished patients with various types of hepatitis from patients with other diseases by performing multivariate analysis using a multiple logistic regression model including the levels of AST and ALT, which are liver function markers in serum, and $\gamma$-Glu-X peptides.
This discovery enabled rapid identification of a normal person and liver diseases such as drug induced liver injury, an asymptomatic hepatitis B carrier, an asymptomatic hepatitis C carrier, chronic hepatitis B, chronic hepatitis C, and liver cancer by measuring the concentrations of $\gamma$-Glu-X peptides and the levels of AST and ALT in blood.
Furthermore, the above-mentioned discovery may be applied to the distinguishment among a nonalcoholic fatty liver disease (NAFLD), nonalcoholic steatohepatitis (NASH), and simple steatosis (SS).
The present invention was achieved based on the above-mentioned finding. The present invention provides a liver disease marker for detecting a toxic electrophile and active oxygen (oxidative stress) in a mammalian tissue, wherein the marker is a $\gamma$-Glu-X (X represents an amino acid) peptide.
The present invention also provides a liver disease marker for identifying a normal person, wherein the above-mentioned liver disease marker is selected from the group consisting of $\gamma$-Glu-Phe, $\gamma$-Glu-Ser, $\gamma$-Glu-Thr, $\gamma$-Glu-Gly, and $\gamma$-Glu-Glu.
The present invention also provides a liver disease marker, wherein the above-mentioned liver disease marker is a combination of a plurality of $\gamma$-Glu-X (X represents an amino acid) peptides. Here, the above-mentioned combination may be selected by multiple logistic regression analysis.
The present invention also provides a liver disease marker for identifying drug-induced hepatitis, wherein the above-mentioned liver disease marker is a combination including at least $\gamma$-Glu-Thr, $\gamma$-Glu-Leu, $\gamma$-Glu-His, and $\gamma$-Glu-Phe.
The present invention also provides a liver disease marker for identifying liver cancer, wherein the above-mentioned liver disease marker is a combination including at least $\gamma$-Glu-Val, $\gamma$-Glu-Thr, $\gamma$-Glu-Leu, $\gamma$-Glu-Phe, and $\gamma$-Glu-Tyr.
The present invention also provides a liver disease marker for identifying an asymptomatic hepatitis B carrier, wherein the above-mentioned liver disease marker is a combination including at least $\gamma$-Glu-Val, $\gamma$-Glu-Gln, $\gamma$-Glu-His, and $\gamma$-Glu-Phe.
The present invention also provides a liver disease marker for identifying chronic hepatitis B, wherein the above-mentioned liver disease marker includes at least $\gamma$-Glu-Lys.
The present invention also provides a liver disease marker for identifying an asymptomatic hepatitis C carrier, wherein the above-mentioned liver disease marker is a combination including at least AST, $\gamma$-Glu-Gly, and $\gamma$-Glu-Phe.
The present invention also provides a liver disease marker for identifying chronic hepatitis C, wherein the above-mentioned liver disease marker is a combination including at least $\gamma$-Glu-Ser, $\gamma$-Glu-Phe, and $\gamma$-Glu-Tyr.
The present invention also provides a liver disease marker, wherein the above-mentioned liver disease marker is used for distinguishing between a nonalcoholic fatty liver disease (NAFLD), nonalcoholic steatohepatitis (NASH), and simple steatosis (SS).

The present invention also provides a method for measuring a liver disease marker, wherein a γ-Glu-X (X represents an amino acid) peptide in a sample is measured as a liver disease marker.

The present invention also provides an apparatus for measuring a liver disease marker, the apparatus including means for preparing a test sample suitable for analysis from a collected sample and analysis means for measuring a γ-Glu-X (X represents an amino acid or an amine) peptide in the test sample as a liver disease marker.

The present invention also provides a method for assaying a pharmaceutical preparation, the method including the steps of: measuring a concentration of any of the above-mentioned liver disease biomarkers in human blood collected before and after administration of the pharmaceutical preparation; and comparing the measurement results between the blood before administration of the above-mentioned pharmaceutical preparation and the blood after administration thereof.

The present invention also provides a method for assaying a pharmaceutical preparation, the method including the steps of: measuring a concentration of any of the above-mentioned liver disease markers in blood collected from a first group consisting of one or more individuals that received the pharmaceutical preparation and blood collected from a second group consisting of one or more individuals that did not receive the pharmaceutical preparation; and comparing the concentrations of the measured liver disease marker between the first group and the second group.

The present invention also provides a method for measuring a liver disease marker, the method including measuring a γ-Glu-X (X represents an amino acid or an amine) peptide in an organ collected from a non-human mammal.

The present invention also provides a method for measuring a liver disease marker, wherein the organ is a liver.

Furthermore, a method for diagnosing a liver disease according to the present invention includes the steps of: collecting blood from one or more human individuals to be diagnosed; measuring a concentration of a marker of the present invention in the collected blood by any of the above-mentioned measuring methods; and comparing the concentration of the marker with that in blood from one or more normal individuals.

A method for diagnosing a toxic side effect caused by an electrophile property of a pharmaceutical preparation (oxidative stress generated by administration of the pharmaceutical preparation) according to the present invention includes the steps of: collecting blood from a human individual before and after administration of the pharmaceutical preparation; measuring a concentration of a marker of the present invention in the collected blood by any of the above-mentioned measuring methods; and comparing the concentration of the marker with that in blood from one or more normal individuals. Here, the pharmaceutical preparation may be of any type.

In the above-mentioned methods, the step of measuring the concentration of a marker includes both measuring separately each of the blood samples collected from individuals and measuring a pool of blood collected from a plurality of individuals. The step of comparing the measured concentration of the marker also includes both comparing each of the concentrations obtained from the respective measurements one by one and comparing a cumulative total or a mean value of the concentrations obtained from the respective measurements.

A mammal in which a marker may be used to detect oxidative stress in its tissue is not limited and may be any mammal as long as the mammal experiences oxidative stress in its tissue and a marker of the present invention may be measured in its blood. The mammal is preferably a human.

Although the mammal from which blood used for this diagnosing method is collected is not particularly limited, a mammal whose blood contains at least one of the above-mentioned markers is preferred. Rodents such as a mouse and a rat, a human, a monkey, and a dog are more preferred.

ADVANTAGEOUS EFFECTS OF INVENTION

According to the present invention, a normal person and liver diseases such as drug induced liver injury, an asymptomatic hepatitis B carrier, an asymptomatic hepatitis C carrier, chronic hepatitis B, chronic hepatitis C, liver cancer, a nonalcoholic fatty liver disease (NAFLD), nonalcoholic steatohepatitis (NASH), and simple steatosis (SS) can be identified rapidly by measuring the concentrations of γ-Glu-X peptides and the levels of AST and ALT in blood.

BRIEF DESCRIPTION OF DRAWINGS

Fig. 1 is a diagram schematically showing a mechanism in which ophthalmic acid is biosynthesized in the presence of an electrophile and active oxygen (oxidative stress).
Fig. 2 is a diagram showing the comparison of the LC-MS measurement results of γ-Glu-X (X represents an amino acid) peptides in the sera from a normal person and a liver cancer patient.
Fig. 3 is a diagram showing the comparison of the measurement results of AST, ALT, and γ-Glu-X peptides in the sera from a normal person and patients with various types of hepatitis.

Fig. 4 is a diagram showing the accuracy of a screening test for a normal status and various liver diseases using AST, ALT, and γ-Glu-X peptides.

Fig. 5 is a diagram showing the comparison of the concentrations of γ-Glu-X peptides in the sera from a liver cancer patient and a gastric cancer patient.

Fig. 6 is a diagram showing the comparison of the quantification results of γ-Glu-X and γ-Glu-X-Gly in the livers of the mice that received BSO or DEM.

Fig. 7 is a diagram schematically showing a mechanism in which γ-Glu-X peptides are biosynthesized in patients with various liver injuries.

MODES FOR IMPLEMENTING THE INVENTION

Hereinbelow, the embodiments of the present invention will be described in detail.

As described above, many liver injuries such as hepatitis, hepatic cirrhosis, and liver cancer are known to be closely related to oxidative stress. Thus, concentrations of ophthalmic acid were measured using capillary electrophoresis-time-of-flight mass spectrometry (CE-TOFMS) in the sera from 10 normal people (C), 31 patients with drug induced liver injury (DI), 8 asymptomatic hepatitis B carriers (AHB), 8 asymptomatic hepatitis C carriers (AHC), 10 patients with chronic hepatitis B (CHB), 21 patients with chronic hepatitis C (CHB), and 14 patients with liver cancer (HCC). However, different substances were found to have increased predominantly in the hepatitis patients and all of these substances were identified as γ-Glu-X peptides (note: X represents an amino acid or an amine).

1. Extraction of Metabolites from Sera

The serum (100 μl) collected from a normal person and patients with various types of hepatitis were added into 900 μl of methanol containing a standard substance to inactivate an enzyme, thereby stopping enhancement of metabolism. After 400 μl of ultrapure water and 1000 μl of chloroform were added, the mixture was centrifuged at 4,600 g for 5 minutes at 4°C. After allowing to stand, 750 μl of separated water-methanol phase was passed through a 5 kDa molecular weight cutoff filter for centrifugal ultrafiltration for deproteinization. The filtrate was lyophilized and 50 μl of Milli-Q water was added thereto. The mixture was subjected to a CE-TOFMS measurement and an LC-MS measurement.

2. Measurement of Metabolites in Sera by Capillary Electrophoresis-Mass Spectroscope (CE-TOFMS)

Low molecular weight metabolic products in the sera from a normal person and a hepatitis patient were measured simultaneously using a CE-TOFMS.

Analysis Conditions for CE-TOFMS a. Analysis Conditions for Capillary Electrophoresis-Electrophoresis (CE)

A fused silica capillary (internal diameter: 50 μm, external diameter: 350 μm, and full length: 100 cm) was used as a capillary. 1M formic acid (pH: approximately 1.8) was used as a buffer solution. Measurement was performed at an applied voltage of +30 kV and at a capillary temperature of 20°C. A sample was injected by pressurization at 50 mbar for 3 seconds (about 3 nl).

b. Analysis Conditions for Time-of-Flight Mass Spectrometer (TOFMS)

Positive ion mode was employed. An ionization voltage, a fragmentor voltage, a skimmer voltage, and an OctRFV voltage were set at 4 kV, 75 V, 50 V, and 125 V, respectively. Nitrogen was used as dry gas, with the temperature set at 300°C and the pressure set at 10 psig. Fifty percent methanol solution was used as sheath fluid. Reserpine (m/z 609.2807) for mass calibration was mixed into the methanol solution to a final concentration of 0.5 μM and the resultant solution was fed at 10 μl/min. Using the mass numbers of reserpine (m/z 609.2807) and an adduct ion of methanol (m/z 83.0703), all the obtained data were automatically calibrated.

3. Measurement of γ-Glu-X Peptides in Sera by Liquid Chromatography-Mass Spectroscope (LC-MSMS)

To achieve a sensitive measurement, γ-Glu-X peptides in sera were measured using a LC-MSMS.

a. Analysis Conditions for Liquid Chromatography (LC)

Develosil RPAQUEOUS-AR-3 (2 mm (internal diameter) x 100 mm (length), 3 μm) from Nomula Chemical Co.

was used as a column for separation, and a column oven was set at 30°C. One microliter of sample was injected into the column. A mobile phase A was 0.5% formic acid and a mobile phase B was acetonitrile. Gamma-Glu-X peptides were separated by an elution method using a gradient of 0% (0 min)-1% (5min)-10% (15min)-99% (17min)-99% (19min) B solution, at a flow rate of 0.2 ml/min.

b. Analysis Conditions for Triple Quadrupole Mass Spectrometer (QqQMS)

An API3000 triple quadrupole mass spectrometer from Applied Biosystem was used for measurement in MRM mode in positive ion mode. The parameters of the mass spectrometer were shown below:

ionspray voltage: 5.5 kV
nebulizer gas pressure: 12 psi
curtain gas pressure: 8 psi
collision gas: 8 unit
temperature of nitrogen gas: 550°C

The MRM parameters optimized for measuring the γ-Glu-X peptides are shown in Table 1.

[Table 1]

| Peptide | Q1 (m/z) | Q3 (m/z) | Declustering Voltage (V) | Focusing Voltage (V) | Collision Energy (V) | Output Voltage of Collision Cell (V) |
|---|---|---|---|---|---|---|
| γ-Glu-Gly | 205 | 84 | 11 | 60 | 31 | 4 |
| γ-Glu-Ala | 219 | 90 | 26 | 100 | 17 | 6 |
| γ-Glu-Ser | 235 | 106 | 36 | 90 | 17 | 18 |
| γ-Glu-Val<br>γ-Glu-Norvaline | 247 | 118 | 26 | 120 | 17 | 8 |
| γ-Glu-Thr<br>γ-Glu-Homoserine | 249 | 120 | 51 | 80 | 17 | 8 |
| γ-Glu-Taurine | 255 | 126 | 46 | 170 | 19 | 8 |
| γ-Glu-Ile<br>γ-Glu-Leu<br>γ-Glu-Norleucine | 261 | 132 | 31 | 150 | 17 | 8 |
| γ-Glu-Asn or γ-Glu-Gly-Gly | 262 | 133 | 16 | 50 | 17 | 22 |
| γ-Glu-O-Ornithine | 262 | 133 | 31 | 90 | 17 | 8 |
| γ-Glu-Asp | 263 | 134 | 31 | 150 | 17 | 8 |
| γ-Glu-Homocysteine | 265 | 136 | 36 | 140 | 17 | 8 |
| γ-Glu-Gln<br>γ-Glu-Ala-Gly<br>γ-Glu-Lys | 276 | 147 | 31 | 100 | 17 | 10 |
| γ-Glu-Glu | 277 | 148 | 26 | 150 | 17 | 10 |
| γ-Glu-M et | 279 | 150 | 36 | 50 | 17 | 6 |
| γ-Glu-His | 285 | 156 | 21 | 110 | 17 | 8 |
| γ-Glu-Ser-Gly | 292 | 163 | 26 | 170 | 17 | 14 |
| γ-Glu-Phe | 295 | 166 | 41 | 220 | 17 | 14 |
| γ-Glu-Val-Gly<br>γ-Glu-Norvaline-Gly | 304 | 175 | 31 | 100 | 17 | 5 |
| γ-Glu-Arg | 304 | 175 | 46 | 110 | 21 | 12 |
| γ-Glu-Citrulline | 305 | 159 | 23 | 110 | 19 | 8 |
| γ-Glu-Thr-Gly | 306 | 177 | 31 | 100 | | 10 |
| γ-Glu-Homoserine-Gly | 306 | 177 | 31 | 100 | 17 | 10 |
| γ-Glu-Tyr | 311 | 182 | 56 | 70 | 19 | 10 |

(continued)

| Peptide | Q1 (m/z) | Q3 (m/z) | Declustering Voltage (V) | Focusing Voltage (V) | Collision Energy (V) | Output Voltage of Collision Cell (V) |
|---|---|---|---|---|---|---|
| γ-Glu-Ile-Gly<br>γ-Glu-Leu-Gly | 318 | 189 | 31 | 100 | 17 | 5 |
| γ-Glu-Asn-Gly | 319 | 173 | 41 | 170 | 19 | 18 |
| γ-Glu-Asp-Gly | 320 | 191 | 26 | 130 | 17 | 10 |
| γ-glu-Homocysteine-Gly | 322 | 193 | 26 | 150 | 17 | 12 |
| γ-Glu-Gln-Gly | 333 | 84 | 16 | 70 | 47 | 4 |
| γ-Glu-Glu-Gly | 334 | 187 | 21 | 90 | 23 | 14 |
| γ-Glu-Trp | 334 | 188 | 41 | 200 | 23 | 14 |
| γ-Glu-Tyr-Gly | 368 | 136 | 31 | 180 | 27 | 12 |
| Ophthalmate | 290 | 169 | 11 | 80 | 15 | 8 |
| GSSG | 307 | 130 | 26 | 140 | 19 | 8 |
| GSH | 308 | 179 | 36 | 130 | 17 | 10 |

4. Search and Evaluation of Liver Injury Biomarkers

Fig. 2 shows the LC-MS measurement results of γ-Glu-X peptides in the sera from a normal person and a liver cancer (HCC) patient. Many of the γ-Glu-X peptides were found to have increased in the liver cancer patient (HCC) compared to the normal person. The concentrations of the γ-Glu-X peptides were also significantly higher in a patient with other liver injuries than in a normal person.

Fig. 3 shows the measurement results of an AST level, an ALT level, and γ-Glu-X peptides in the sera from a normal person (C) and patients with various types of hepatitis. In this figure, the arrows show the maximum and the minimum, the top of the box corresponds to a measurement ranked in the top 25% and the bottom of the box corresponds to a measurement ranked in the bottom 75%, and the transverse line in the box shows a median.

Although the AST level and the ALT level, which are conventional liver function test values, had increased in drug-induced hepatitis (DI), chronic hepatitis B (CHB), and chronic hepatitis C (CHC), their levels in the other types of hepatitis were not significantly different from the levels in the normal person.

However, a patient with drug-induced hepatitis (DI) showed a higher level of γ-Glu-X peptides such as γ-Glu-Ser and γ-Glu-Thr than the normal person (C), and patients with the other types of hepatitis showed an even higher level of γ-Glu-X peptides. Particularly, patients with asymptomatic hepatitis B (AHB), asymptomatic hepatitis C (AHC), or liver cancer (HCC) also showed a high level of γ-Glu-X peptides. Furthermore, it was observed in more detailed investigation that some γ-Glu-X peptides were found at a higher level in asymptomatic hepatitis C (AHC) than in asymptomatic hepatitis B (AHB), and in chronic hepatitis B (CBC) than in asymptomatic hepatitis B (AHB), and some γ-Glu-X peptides showed a tendency to decrease as hepatitis C progressed from asymptomatic hepatitis C (AHC) to chronic hepatitis C (CHC), and further to liver cancer (HCC) even if all of them are derived from the same hepatitis C.

As described above, the blood concentrations of AST, ALT, and each of the γ-Glu-X peptides were different in each of the diseases. Therefore, we expected that the diseases could be classified by using the measurements of these components. Then, multiple logistic regression analysis, which is a methodology for multivariate analysis, was performed to select a biomarker for distinguishing each liver disease. The result is shown in Table 2.

[Table 2]

| Groups | Markers | Parameters | Standard Errors | 95% CI Lower Limits | 95% CI Upper Limits | Odds Ratios | 95% CI Lower Limits | 95% CI Upper Limits | p-Values |
|---|---|---|---|---|---|---|---|---|---|
| **C** | (Intercept) | 3.61 | 1.48 | 1.00 | 6.90 | - | | - | 0.0147 |
| | γ-Glu-Phe | -34.5 | 10.9 | -59.9 | -16.5 | $1.07 \times 10^{-15}$ | $9.26 \times 10^{-27}$ | $6.89 \times 10^{-8}$ | 0.0016 |
| **DI** | (Intercept) | 27.3 | 12.6 | 10.6 | 65.4 | | - | - | 0.0303 |
| | ALT | 0.0401 | 0.0196 | 0.0139 | 0.101 | 1.04 | 1.01 | 1.11 | 0.0403 |
| | γ-Glu-Thr | 21.5 | 10.5 | 7.30 | 52.7 | $2.22 \times 10^{9}$ | $1.49 \times 10^{-3}$ | $7.50 \times 10^{22}$ | 0.0396 |
| | γ-Glu-Leu | -45.3 | 23.0 | $-1.23 \times 10^{2}$ | -15.7 | $2.05 \times 10^{-20}$ | 0.00 | $1.57 \times 10^{-7}$ | 0.0484 |
| | γ-Glu-His | -32.8 | 16.7 | -80.3 | -10.8 | $5.50 \times 10^{-15}$ | $1.29 \times 10^{-35}$ | $1.98 \times 10^{-5}$ | 0.0489 |
| | γ-Glu-Phe | 41.5 | 19.1 | 15.4 | 98.7 | $1.08 \times 10^{18}$ | $4.72 \times 10^{6}$ | $7.36 \times 10^{42}$ | 0.0293 |
| **AHB** | (Intercept) | 2.86 | 2.15 | -0.948 | 7.87 | - | - | | 0.1831 |
| | ALT | -0.0665 | 0.0295 | -0.146 | -0.0204 | 0.936 | 0.864 | 0.980 | 0.0245 |
| | γ-Glu-Val | 4.06 | 1.64 | 1.20 | 7.86 | 58.2 | 3.31 | $2.60 \times 10^{3}$ | 0.0133 |
| | γ-Glu-Gln | 0.402 | 0.140 | 0.171 | 0.742 | 1.49 | 1.19 | 2.10 | 0.0041 |
| | γ-Glu-His | -6.37 | 3.24 | -13.5 | -0.265 | $1.72 \times 10^{-3}$ | $1.44 \times 10^{-6}$ | 0.767 | 0.0494 |
| | γ-Glu-Phe | -24.3 | 10.4 | -48.8 | -6.32 | $2.73 \times 10^{-11}$ | $6.20 \times 10^{-22}$ | $1.80 \times 10^{-3}$ | 0.0198 |
| **CHB** | (Intercept) | -4.15 | 0.794 | -6.01 | -2.80 | - | - | - | <.0001 |
| | AST | $5.12 \times 10^{-3}$ | $2.56 \times 10^{-3}$ | $1.23 \times 10^{-3}$ | 0.0115 | 1.01 | 1.00 | 1.01 | $4.54 \times 10^{-2}$ |
| | γ-Glu-Lys | 0.609 | 0.242 | 0.156 | 1.13 | 1.84 | 1.17 | 3.09 | 0.0118 |
| **AHC** | (Intercept) | 2.29 | 2.34 | -1.52 | 8.05 | | | - | 0.3277 |
| | AST | -0.209 | 0.112 | -0.505 | -0.0561 | 0.811 | 0.603 | 0.945 | 0.0629 |
| | γ-Glu-Gly | 0.248 | 0.103 | 0.0965 | 0.527 | 1.28 | 1.10 | 1.69 | 0.0158 |
| | γ-Glu-Phe | -8.39 | 5.78 | -23.8 | 0.316 | $2.26 \times 10^{-4}$ | $4.69 \times 10^{-11}$ | 1.37 | 0.1463 |
| **CHC** | (Intercept) | -2.86 | 0.564 | -4.08 | -1.85 | - | | - | <.0001 |
| | γ-Glu-Ser | 0.351 | 0.269 | -0.182 | 0.893 | 1.42 | 0.834 | 2.44 | 0.1917 |
| | γ-Glu-Phe | -3.32 | 2.41 | -8.52 | 1.13 | 0.0361 | $1.99 \times 10^{-4}$ | 3.09 | 0.1681 |
| | γ-Glu-Tyr | 3.91 | 1.75 | 0.723 | 7.66 | 50.1 | 2.06 | $2.11 \times 10^{3}$ | 0.0256 |
| **HCC** | (Intercept) | -3.62 | 1.43 | -7.09 | -1.19 | - | - | - | 0.0115 |
| | AST | 0.146 | 0.0555 | 0.0516 | 0.279 | 1.16 | 1.05 | 1.32 | 0.0085 |
| | ALT | -0.215 | 0.0714 | -0.390 | -0.101 | 0.807 | 0.677 | 0.904 | 0.0027 |
| | γ-Glu-Val | 2.75 | 1.42 | 0.0366 | 5.89 | 15.6 | 1.04 | $3.60 \times 10^{2}$ | 0.0536 |
| | γ-Glu-Thr | -5.96 | 3.46 | -13.8 | 0.247 | $2.58 \times 10^{-3}$ | $1.03 \times 10^{14}$ | 1.28 | 0.0846 |

(continued)

| Groups | Markers | Parameters | Standard Errors | 95% CI | | Odds Ratios | 95% CI | | p-Values |
|---|---|---|---|---|---|---|---|---|---|
| | | | | Lower Limits | Upper Limits | | Lower Limits | Upper Limits | |
| | γ-Glu-Leu | -7.15 | 3.20 | -14.8 | -1.88 | $7.87 \times 10^{-4}$ | $3.64 \times 10^{-7}$ | 0.152 | 0.0253 |
| | γ-Glu-Phe | 14.0 | 8.73 | -0.941 | 34.4 | $1.18 \times 10^{6}$ | 0.390 | $8.43 \times 10^{14}$ | 0.1094 |
| | γ-Glu-Tyr | 18.9 | 6.74 | 8.06 | 35.2 | $1.69 \times 10^{8}$ | $3.15 \times 10^{3}$ | $1.90 \times 10^{15}$ | 0.0049 |

In multiple logistic regression analysis, a regression equation (1) for p is solved:

$$\ln (p/1 - p) = b_0 + b_1x_1 + b_2x_2 + b_3x_3 + ... + b_kx_k \quad (1),$$

wherein explanatory variables of the number k, such as $x_1$, $x_2$, $x_3$, ..., $x_k$, are used for calculating ratio p, which is an objective variable. Values of the parameters in Table 2 are specific values for $b_0$, $b_1$ ...$b_k$ in the equation (1). The (Intercept) represents the value of a constant term ($b_0$).

To calculate probabilities for each case, for example in the case of a drug-induced hepatitis (DI) group, a specific value is obtained by assigning the value of the (Intercept) parameter in the table, 27.3, to $b_0$, the value of ALT parameter, 0.0401, to $b_1$, the value of γ-Glu-Thr parameter, 21.5, to $b_2$, ..., the value of γ-Glu-Phe parameter, 41.5, to $b_5$, and a quantified amount of ALT to $x_1$, a quantified amount of γ-Glu-Thr to $x_2$, ..., a quantified amount of γ-Glu-Phe to $x_5$. Estimated standard errors and 95% confidence intervals of the parameters are also indicated in the table.

Based on the result of this analysis, candidate biomarkers were discovered that allowed for a selective distinguishment of many types of hepatitis patients as well as normal persons (C). For example, it was found that the biomarker for identifying a normal person (C) was γ-Glu-Phe and a normal person could be distinguished from patients with other liver diseases by the level of γ-Glu-Phe. Furthermore, Fig. 3 indicates that γ-Glu-Ser, γ-Glu-Thr, γ-Glu-Gly, and γ-Glu-Glu can also be used.

The biomarkers of drug-induced hepatitis (DI) are ALT, γ-Glu-Thr, γ-Glu-Leu, γ-Glu-His, and γ-Glu-Phe. When, for example, logistic regression analysis using a combination of these biomarkers is performed and the value of p is more than a predetermined value, for example, 0.5, the disease can be identified as drug-induced hepatitis and be distinguished from other liver diseases. Among these biomarkers, the level of γ-Glu-Phe whose odds ratio is the highest and greater than 1 contributes best to identification of drug-induced hepatitis (DI), wherein the odds ratio explains how much the probability p changes when a quantified amount $x_1$ increases by one. On the other hand, γ-Glu-Leu and γ-Glu-His whose odds ratios are near 0 contribute to identification of diseases other than drug-induced hepatitis (non-DI), and thus, an increase in these substances indicates that the disease is non-DI. The odds ratio of ALT is 1.04, which is close to 1.0, meaning that there is no change of p in association with an increase in a variable. Therefore, ALT contributes little and may be omitted.

The biomarkers of liver cancer (HCC) are AST, ALT, γ-Glu-Val, γ-Glu-Thr, γ-Glu-Leu, γ-Glu-Phe, and γ-Glu-Tyr. When, for example, logistic regression analysis using a combination of these biomarkers is performed and the value of p is more than a predetermined value, for example, 0.5, the disease can be identified as liver cancer and be distinguished from other liver diseases. Among these biomarkers, the level of γ-Glu-Tyr whose odds ratio is the highest and greater than 1 contributes best to identification of liver cancer (HCC). On the other hand, γ-Glu-Thr and γ-Glu-Leu whose odds ratios are near 0 contribute to identification of diseases other than liver cancer (non-HCC), and thus, an increase in these substances indicates that the disease is non-HCC. The odds ratios of AST and ALT are 1.16 and 0.807, respectively, which are close to 1, and they contribute little. Therefore, AST and ALT may be omitted.

Similarly, for the other diseases, their respective candidate biomarkers were discovered as shown in Table 2. The biomarkers of an asymptomatic hepatitis B carrier (AHB) were ALT, γ-Glu-Val, γ-Glu-Gln, γ-Glu-His, and γ-Glu-Phe, the biomarkers of chronic hepatitis B (CHB) were AST and γ-Glu-Lys, the biomarkers of an asymptomatic hepatitis C carrier (AHC) were AST, γ-Glu-Gly, and γ-Glu-Phe, and the biomarkers of chronic hepatitis C (CHC) were γ-Glu-Ser, γ-Glu-Phe, and γ-Glu-Ty. Among these, the biomarkers whose odds ratios are near 1, for example, ALT (1.04) for identifying AHB and AST (1.01) for identifying CHB may be omitted.

The minimal combination of required markers was searched by performing stepwise variable selection. That is, a forward selection method was employed, which involves starting with no variables and repeating additions of more significant variables and exclusions of less significant variables, thereby finding the most suitable combination. In the current study, the p value of 0.25 was used as a threshold value for both additions and eliminations.

Regarding the contribution ratios of these biomarkers, the accuracy of the logistic model may also be further increased by adding the data of cases and allowing the model to restudy the data, and modifying the combination of the biomarkers for each identification and the coefficients of the logistic model.

Subsequently, the accuracy of each of the screening tests for the liver diseases using these biomarkers was evaluated using a receiver operating characteristic curve (receiver operating curve: ROC curve). The results are shown in Fig. 4 and Table 3.

[Table 3]

| Groups | Sensitivity (%) | Specificity (%) | Accuracy (%) | ROC | 95% CI Lower Limits | 95% CI Upper Limits | P-Values |
|---|---|---|---|---|---|---|---|
| C | 100 | 78.0 | 89.0 | 0.939 | 0.886 | 0.991 | $< 1.00 \times 10^{-4}$ |
| DI | 100 | 95.7 | 97.9 | 0.998 | 0.993 | 1.00 | $< 1.00 \times 10^{-4}$ |
| AHB | 75.0 | 98.9 | 87.0 | 0.933 | 0.852 | 1.01 | $< 1.00 \times 10^{-4}$ |
| CHB | 100 | 57.1 | 78.6 | 0.859 | 0.749 | 0.969 | $2.08 \times 10^{-4}$ |
| AHC | 87.5 | 89.2 | 88.4 | 0.944 | 0.882 | 1.01 | $< 1.00 \times 10^{-4}$ |
| CHC | 95.2 | 63.7 | 79.5 | 0.811 | 0.726 | 0.895 | $< 1.00 \times 10^{-4}$ |
| HCC | 92.3 | 97.7 | 95.0 | 0.974 | 0.937 | 1.01 | $< 1.00 \times 10^{-4}$ |

Fig. 4 and Table 3 show the accuracy of the multiple logistic regression model that distinguishes a certain disease group from all the other disease groups. For example, in the case of drug-induced hepatitis (DI), DI is distinguished from all the diseases other than DI. As Fig. 4 and Table 3 show, the area under the ROC curve (area under the receiver-operating curve: AUC) was 0.81 to 0.99 in all the diseases, verifying that each of the screening tests for the liver diseases using these biomarkers allowed a highly accurate identification of each disease. In particular, it turned out that a normal person (C: AUC = 0.939), drug-induced hepatitis (DI: AUC = 0.998), an asymptomatic hepatitis B carrier (AHB: AUC = 0.933), an asymptomatic hepatitis C carrier (AHC: AUC = 0.944), and liver cancer (HCC: AUC = 0.937) were identified with a very high accuracy by this method.

5. Evaluation of γ-Glu-X Peptides in Other Diseases

We determined whether γ-Glu-X peptides were increased in other diseases. Fig. 5 shows the concentrations of γ-Glu-X peptides in the sera from a liver cancer patient (HCC) and a gastric cancer patient (GC). In the gastric cancer patient, the concentrations of γ-Glu-X peptides were equivalent to those of a normal person and there was no increase in the γ-Glu-X peptides which was seen in the liver cancer patient. (Note: it is reported that infection with Helicobacter pylori causes gastric cancer and suppresses oxidative stress (reference 10). It is suspected that the concentrations of γ-Glu-X peptides are low since gastric cancer is not exposed to oxidative stress).

The concentrations of γ-Glu-X peptides were also low in the blood from a diabetic patient. Thus, the increase of γ-Glu-X peptides in blood was observed in a liver disease specific manner.

6. Elucidation of Biosynthesis Mechanism of γ-Glu-X Peptides

The biosynthesis mechanism of γ-Glu-X peptides was elucidated using a mouse. As shown in Fig. 1(B), the following biosynthesis mechanism of γ-Glu-X peptides was found. That is, the oxidative stress condition caused by active oxygen and electrophiles causes depletion of glutathione, which is used for removing these substances, and this depletion leads to activation of γ-glutamyl cysteine synthase (GCS). Then, γ-Glu-X dipeptides and γ-Glu-X-Gly tripeptides are biosynthesized by using various amino acids as substrates (starting materials). The experimental procedures are described below.

a. Administration of GCS Inhibitor, BSO, and GCS Activator, DEM, to Mice

Male mice were fasted overnight and anesthetized by an intraperitoneal injection of pentobarbital sodium (60 mg/Kg of body weight). Then, the mice were intraperitoneally injected with BSO, which is a γ-glutamyl cysteine synthase (GCS) inhibitor, and diethylmaleate (DEM), which is an electrophile (GCS activator), both at a dose of 4 mmol/kg of body weight (888 mg of BSO, 688 mg of DEM). The mouse as a healthy subject was intraperitoneally injected with physiological saline. Liver specimens (about 300 mg) were excised from the mice 1 hour after administration (5 times each).

b. Extraction of Metabolites from the Livers

The liver specimens (about 300 mg) excised from the mice were immediately placed in 1 ml of methanol containing an internal standard substance and were homogenized to inactivate an enzyme, thereby stopping enhancement of metabolism. After 500 μl of pure water was added to the mixture, 300 μl of solution was taken out. To this solution, 200 μl of chloroform was added. After thorough stirring, the solution was further centrifuged at 15000 rpm for 15 minutes at 4°C. After allowing to stand, 300 μl of separated water-methanol phase was passed through a 5 kDa molecular weight cutoff filter for centrifugal ultrafiltration to deproteinize it. The filtrate was lyophilized and 50 μl of Milli-Q water was added thereto. The mixture was subjected to a CE-TOFMS measurement.

c. Identification Results of γ-Glu-X Peptides and γ-Glu-X-Gly Peptides as Biomarkers That Are Indicators of Oxidative Stress

Fig. 6 shows a part of the measurement results of amino acids, γ-Glu-X peptides, and γ-Glu-X-Gly peptides in the livers and sera from the mice after administration of physiological saline (a healthy subject), BSO, or DEM. The left part of Fig. 6 shows the quantification results of amino acids (X), γ-Glu-X peptides, and γ-Glu-X-Gly peptides detected in the livers from the mice that received each of the reagents. The right part of Fig. 6 shows the quantification results of amino acids (X), γ-Glu-X peptides, and γ-Glu-X-Gly peptides in the sera from the mice.

For example, the graphs on the top show the results of Cys, γ-Glu-Cys, and γ-Glu-Cys-Gly (glutathione). The amount of glutathione in the livers was decreased rapidly in the BSO-administered mouse and the DEM-administered mouse compared to the healthy mouse (In the BSO-administered mouse, glutathione is decreased because of inhibition of γ-glutamyl cysteine synthase. In the electrophilic DEM-administered mouse, glutathione is decreased since it is consumed for detoxication). No glutathione related substances were detected in the sera.

The method described below was used to confirm that the detected γ-Glu-X peptides and γ-Glu-X-Gly peptides were synthesized in a glutathione-biosynthesis pathway. As shown in Fig. 7, if these peptides were synthesized in the glutathione-biosynthesis pathway, the γ-Glu-X peptides and γ-Glu-X-Gly peptides in the liver should be decreased by administration of BSO (since γ-glutamyl cysteine synthase is inhibited) and be increased by administration of electrophilic DEM (since γ-glutamyl cysteine synthase is activated), compared to the healthy subject. As shown in Fig. 6, γ-Glu-X peptidic substances and γ-Glu-X-Gly peptidic substances in the liver other than glutathione-related γ-Glu-Cys, GSH, and γ-Glu-Ser-Gly were decreased by administration of BSO and were increased by administration of DEM compared to the healthy subject. Therefore, it was confirmed that the γ-Glu-X peptidic substances and γ-Glu-X-Gly peptidic substances were certainly generated in the glutathione-biosynthesis pathway.

In short, it was found that these γ-Glu-X peptides and γ-Glu-X-Gly peptides were biosynthesized in the liver when glutathione was decreased because of oxidative stress caused by active oxygen, electrophiles, and the like.

d. Tracing of the Biosynthesis Pathway Using Threonine Isotope

Furthermore, 13C, 15N isotope-labeled threonine (Thr) was administered to a mouse intraperitoneally, and APAP, which produces an electrophile and thereby gives oxidative stress, was given to the mouse. γ-Glu-Thr and γ-Glu-Thr-Gly containing 13C, 15N-labeled Thr were detected in the murine liver and it was confirmed that γ-Glu-Thr and γ-Glu-Thr-Gly were certainly biosynthesized from Thr under the oxidative stress condition.

On the other hand, regarding the substances in the murine serum, γ-Glu-X peptides and γ-Glu-X-Gly peptides that were decreased by administration of BSO and were increased by administration of an electronic substance, DEM, compared to the normal state were only γ-Glu-2AB and ophthalmic acid (γ-Glu-2AB-Gly) (Fig. 6). Therefore, it is expected that, in the case of a mouse, only γ-Glu-2AB and ophthalmic acid are increased in the blood when glutathione is decreased because of the oxidative stress caused by electrophiles or the like.

However, when the sera from patients with various types of hepatitis were measured, the concentrations of the other γ-Glu-X peptides were higher than those of γ-Glu-2AB and ophthalmic acid. This difference occurs presumably since the substrate concentration, the substrate specificity and activity of a metabolic enzyme, the type, function, and level of expression of a transporter, and the like are different between organism species.

The diagnosis method according to the present invention based on the measurement of γ-Glu-X peptides in serum can also apply to diagnosis of various liver injuries such as hepatic cirrhosis, a nonalcoholic fatty liver disease (NAFLD), nonalcoholic hepatitis (NASH), and simple steatosis (SS). In this specification, exemplary combinations of AST, ALT, and γ-Glu-X peptides, which were candidate markers of various liver injuries, were described; however, the combinations of candidate biomarkers are not limited thereto and may be changed through a future further close investigation using multiple samples.

Although a LC-MS method was used for measuring γ-Glu-X peptides in serum in this study, measuring methods are not limited and any analysis method can be used for the measurement. Exemplary measuring methods include gas chromatography (GC), liquid chromatography (LC), capillary electrophoresis (CE), chip LC, chip CE, and GC-MS, LC-MS, and CE-MS methods that combine them with a mass spectrometer (MS), various measuring methods using MS alone, an NMR method, a method of measuring γ-Glu-X peptides after their derivatization to fluorescent substances or UV absorbing substances, a measuring method involving producing an antibody and performing an ELISA method using the antibody, and the like.

The method for assaying a pharmaceutical preparation according to the present invention is a method for measuring the concentration of the marker of the present invention in a mammal, by using the blood collected from the mammal that received the pharmaceutical preparation and the blood collected from the mammal that did not receive the pharmaceutical preparation. Although the mammal from which blood used for this assay method is collected is not particularly limited, a mammal whose blood contains at least one of the above-mentioned markers is preferred. Rodents such as a mouse and a rat, a human, a monkey, and a dog are more preferred.

In the above-mentioned method, when the efficacy of a pharmaceutical preparation as a therapeutic agent for a toxic electrophile and active oxygen (oxidative stress) is examined, the targeted disease to which the pharmaceutical preparation is applied is not limited as long as it is a disease caused by oxidative stress. The targeted disease is similar to the diseases as described above for which a marker is used. Furthermore, when the strength of the oxidative stress generated by administration of a pharmaceutical preparation is examined, the type of the pharmaceutical preparation is not limited in any way. For example, pharmaceutical preparations may include a harmful drug.

The purposes of the assay method according to the present invention are to examine the efficacy of a pharmaceutical preparation as a therapeutic agent for a disease caused by oxidative stress and to examine the strength of the oxidative stress generated by administration of the pharmaceutical preparation; however, in practice, the method may be used in various situations. Although representative examples of use of the assay method according to the present invention are described below, the inventive assay method is not limited thereto.

(1) Assay of Efficacy as Therapeutic Agent

Hereinbelow, exemplary uses in hepatitis of the assay method according to the present invention are described; however, the assay method is not limited to these examples.

(1-1) Medicinal Effect Assay in Specific Individual

For example, the assay method according to the present invention can be used to determine if a therapeutic agent for hepatitis is effective in treating the hepatitis of a specific patient. First, blood is collected from a patient suffering from hepatitis before and after administration of the therapeutic agent for hepatitis to the patient. Then, the concentration of a hepatitis diagnostic marker in the blood is measured. The thus obtained marker concentrations in the blood before and after administration of the therapeutic agent for hepatitis are compared. When the marker concentration in the blood after administration of the therapeutic agent for hepatitis is significantly lower compared to that before administration, one can determine that the therapeutic agent for hepatitis is effective in treating the hepatitis of the patient.

(1-2) General Medicinal Effect Assay

Furthermore, it is also possible to assay general efficacy of the pharmaceutical preparation as a therapeutic agent for hepatitis by applying the assay method according to the present invention to a plurality of human individuals.

For example, by comparing the concentrations of a hepatitis diagnostic marker before and after administration of a therapeutic agent for hepatitis in a plurality of humans suffering from hepatitis, one can examine a universal effect of the substance as a therapeutic agent.

Alternatively, in another embodiment, the effect as a pharmaceutical preparation may be compared between two groups. First, patients suffering from hepatitis are divided into two groups. The patients of one group receive a therapeutic agent for hepatitis, while the patients of the other group do not receive the therapeutic agent or receive a placebo. Blood is collected from the patients of these two groups. Then, the concentration of a hepatitis diagnostic marker in the blood is measured. Furthermore, the concentrations of the marker in the blood obtained by this measurement are compared between the two groups.

Herein, a "group" may include only a single individual or a plurality of individuals, and the number of the individuals of the two groups may be the same or different. The blood collected from the individuals in the same group may be pooled and the marker concentration in the pooled blood may be measured. However, it is preferable to measure the marker concentration in each individual's blood separately.

The comparison of the marker concentrations among groups including a plurality of blood samples may be performed by comparing pairs of blood samples or by comparing among the groups a cumulative total or a mean value of the marker concentrations obtained from the plurality of blood samples belonging to the same group. The plurality of blood samples are for example, blood samples before and after or in the presence or absence of administration of a pharmaceutical preparation. This comparison may be performed using any statistical method well known to those of ordinary skill in the art. As a result of such comparisons, when the marker concentration in the blood after administration of a therapeutic agent is significantly lower compared to that before administration or when the marker concentration in the blood from the group receiving the therapeutic agent is significantly lower compared to that of the group receiving no therapeutic agent, one can determine that the therapeutic agent is effective for the treatment of hepatitis. Moreover, one can determine the level of efficacy by the degree of decrease.

In this way, screening for a therapeutic agent for hepatitis can be achieved by assaying its general efficacy as a therapeutic agent for hepatitis. Furthermore, the strength of medicinal effect of each therapeutic agent for hepatitis can be examined by using a plurality of therapeutic agents for hepatitis and examining therapeutic effects of different concentrations of each of the therapeutic agents for hepatitis, and comparing their different medicinal effects dependent on their concentrations.

(2) Assay of the Strength of Oxidative Stress

Strong oxidative stress leads to a strong expression of a side effect. Therefore, exemplary uses of the assay method according to the present invention for the side effect of a pharmaceutical preparation is described below by way of example; however, the inventive assay method is not limited to these examples.

(2-1) Assay of the Strength of Side Effect in Specific Individual

The assay method according to the present invention can be used to determine if a certain pharmaceutical preparation brings a side effect to a specific mammalian individual. First, blood is collected from the individual before and after administration of the therapeutic pharmaceutical preparation to the individual. Then, the concentration of an oxidative stress assay marker in the blood is measured. The thus obtained marker concentrations in the blood before and after administration of the pharmaceutical preparation are compared. When the marker concentration in the blood after administration of the pharmaceutical preparation is significantly higher compared to that before administration, one can determine that the administered pharmaceutical preparation caused oxidative stress in the individual, causing a side effect in the individual.

(2-2) Assay of General Strength of a Side Effect

Furthermore, it is also possible to assay a general strength of a side effect of a certain pharmaceutical preparation by applying the assay method according to the present invention to a plurality of mammalian individuals.
For example, it is possible to examine a general strength of a side effect of the pharmaceutical preparation by comparing the concentrations of an oxidative stress assay marker before and after administration of the pharmaceutical preparation for treating a disease in a plurality of individuals suffering from the disease.
Alternatively, in another embodiment, the strength of a side effect can be compared between two groups. First, mammals suffering from a disease are divided into two groups. The individuals of one group receive the pharmaceutical preparation for treating the disease, while the individuals of the other group do not receive the pharmaceutical preparation or receive a placebo. Blood is collected from the individuals of these two groups. Then, the concentration of an oxidative stress assay marker in the blood is measured. Furthermore, the concentrations of the markers in the blood obtained by this measurement are compared between the two groups. Herein, a "group" may include only a single individual or a plurality of individuals, and the number of the individuals of the two groups may be the same or different. The blood collected from the individuals in the same group may be pooled and the marker concentration in the pooled blood may be measured. However, it is preferable to measure the marker concentration in each individual’s blood separately.
The comparison of the marker concentrations among groups may be performed by comparing pairs of blood samples or by comparing among the groups a cumulative total or a mean value of the marker concentrations obtained from the plurality of blood samples belonging to the same group. This comparison may be performed using any statistical method well known to those of ordinary skill in the art. As a result of such comparisons, when the marker concentration in the blood after administration of the pharmaceutical preparation is significantly higher compared to that before administration or when the marker concentration in the blood from the group receiving the pharmaceutical preparation is significantly higher compared to that of the group receiving no pharmaceutical preparation, one can determine that the pharmaceutical preparation has a side effect.
In this way, screening for a pharmaceutical preparation with a weak side effect can be achieved by assaying the strength of the side effect of the pharmaceutical preparation. Furthermore, the suitability of each pharmaceutical preparation as a therapeutic agent can be compared by using a plurality of pharmaceutical preparations and examining the pharmaceutical effects of different concentrations of each of the pharmaceutical preparations, and comparing their different side effects dependent on their concentrations.
As described above, the oxidative stress assay marker of the present invention can be used for the assay of a pharmaceutical preparation for treating a liver disease or the assay of the strength of a side effect of a pharmaceutical preparation, diagnosis of a disease, and the like. On that occasion, the assay accuracy and diagnosis accuracy can be increased by using a plurality of markers. Assay methods and diagnosis methods other than the marker of the present invention may also be combined.

INDUSTRIAL APPLICABILITY

The present invention discovered a biomarker, a γ-Glu-X peptide, which indicates the depletion of glutathione caused by oxidative stress that was generated in a living organism. The γ-Glu-X peptide is not only useful as a rapid screening method for patients with various liver injuries, but also can serve as a marker for detecting oxidative stress in a living organism in a wide range of life science fields.

## Claims

1. *In vitro* use of at least one liver disease marker selected from the group of γ-Glu-Gly, γ-Glu-Ala, γ-Glu-Ser, γ-Glu-Val, γ-Glu-Thr, γ-Glu-Taurine, y-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrulline, and γ-Glu-Tyr for detecting oxidative stress in mammalian tissue.

2. The use of at least one liver disease marker according to claim 1, **characterized in that** the marker is the liver disease marker for identifying a normal person, which is selected from the group consisting of γ-Glu-Phe, γ-Glu-Ser, γ-Glu-Thr, γ-Glu-Gly, and γ-Glu-Glu.

3. *In vitro* use of a liver disease marker for detecting oxidative stress in mammalian tissue, **characterized in that** the marker is a combination of a plurality of γ-Glu-X (X represents an amino acid) peptides.

4. The use of a liver disease marker according to claim 3, **characterized in that** the marker is the liver disease marker for identifying drug-induced hepatitis, which is a combination including at least γ-Glu-Thr, γ-Glu-Leu, γ-Glu-His, and γ-Glu-Phe.

5. The use of a liver disease marker according to claim 3, **characterized in that** the marker is the liver disease marker for identifying liver cancer, which is a combination including at least γ-Glu-Val, γ-Glu-Thr, γ-Glu-Leu, γ-Glu-Phe, and γ-Glu-Tyr.

6. The use of a liver disease marker according to claim 3, **characterized in that** the marker is the liver disease marker for identifying an asymptomatic hepatitis B carrier, which is a combination including at least γ-Glu-Val, y-Glu-Gln, γ-Glu-His, and γ-Glu-Phe.

7. The use of a liver disease marker according to claim 1, **characterized in that** the marker is the liver disease marker for identifying chronic hepatitis B, which includes at least γ-Glu-Lys.

8. The use of a liver disease marker according to claim 3, **characterized in that** the marker is the liver disease marker for identifying an asymptomatic hepatitis C carrier, which is a combination including at least AST, γ-Glu-Gly, and γ-Glu-Phe.

9. The use of a liver disease marker according to claim 1, **characterized in that** the marker is the liver disease marker for identifying chronic hepatitis C, which is a combination including at least γ-Glu-Ser, γ-Glu-Phe, and γ-Glu-Tyr.

10. *In vitro* use of a liver disease marker for distinguishing between a nonalcoholic fatty liver disease (NAFLD), nonalcoholic steatohepatitis (NASH), and simple steatosis (SS), **characterized in that** the marker is selected from the group of γ-Glu-Gly, γ-Glu-Ala, γ-Glu-Ser, γ-Glu-Val, γ-Glu-Thr, γ-Glu-Taurine, γ-Glu-Ile, γ-Glu-Asn, γ-Glu-Asp, γ-Glu-Gln, γ-Glu-Lys, γ-Glu-Glu, γ-Glu-Met, γ-Glu-His, γ-Glu-Trp, γ-Glu-Arg, and γ-Glu-Citrulline.

11. An *in vitro* method for detecting oxidative stress, **characterized in that** at least one of γ-Glu-Gly, γ-Glu-Ala, γ-Glu-Ser, γ-Glu-Val, γ-Glu-Thr, γ-Glu-Taurine, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrulline, and γ-Glu-Tyr is measured in a sample.

12. A method for assaying a pharmaceutical preparation, **characterized by** comprising the steps of:

    measuring a concentration of the liver disease biomarker as set forth in any one of claims 1 to 11 in human blood collected before and after administration of the pharmaceutical preparation; and
    comparing the measurement results between the blood before administration of the above-mentioned pharmaceutical preparation and the blood after administration thereof.

13. A method for assaying a pharmaceutical preparation, **characterized by** comprising the steps of:

measuring a concentration of the liver disease marker as set forth in any one of claims 1 to 11 in blood collected from a first group consisting of one or more individuals that received the pharmaceutical preparation and blood collected from a second group consisting of one or more individuals that did not receive the pharmaceutical preparation; and
comparing the concentrations of the measured liver disease marker between the first group and the second group.

14. A method for detecting oxidative stress, **characterized by** measuring one of γ-Glu-Gly, γ-Glu-Ala, γ-Glu-Ser, γ-Glu-Val, γ-Glu-Thr, γ-Glu-Taurine, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrulline, and γ-Glu-Tyr in a liver organ collected from a non-human mammal.

15. A method for detecting hepatitis B or C, the method comprising the steps of:

collecting blood from one or more human individuals;
measuring the concentration of γ-Glu-X in the collected blood; and
comparing the concentration of the marker with that in blood from one or more normal individuals.

16. The method for detecting hepatitis B or C according to claim 15, wherein the hepatitis is an asymptomatic hepatitis, or a chronic hepatitis.

17. The method for detecting hepatitis B or C according to claims 15 or 16, wherein the γ-Glu-X is selected from the group consisting of γ-Glu-Gly, γ-Glu-Ser, γ-Glu-Val, γ-Glu-Thr, γ-Glu-Taurine, γ-Glu-Leu, γ-Glu-Gln, γ-Glu-Lys, γ-Glu-Glu, γ-Glu-His, γ-Glu-Phe, and γ-Glu-Tyr.

## Patentansprüche

1. *In vitro* Verwendung wenigstens eines Markers für Lebererkrankungen, der aus der Gruppe γ-Glu-Gly, γ-Glu-Ala, γ-Glu-Ser, γ-Glu-Val, γ-Glu-Thr, γ-Glu-Taurin, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrullin und γ-Glu-Tyr ausgewählt wird für den Nachweis von oxidativem Stress in Gewebe von Säugetieren.

2. Die Verwendung wenigstens eines Markers für Lebererkrankungen gemäß Anspruch 1, dadurch charakterisiert, dass der Marker der Marker für Lebererkrankungen zur Identifikation einer normalen Person ist, der aus der Gruppe bestehend aus γ-Glu-Phe, γ-Glu-Ser, γ-Glu-Thr, γ-Glu-Gly und γ-Glu-Glu ausgewählt wird.

3. *In vitro* Verwendung eines Markers für Lebererkrankungen zum Nachweis von oxidativem Stress in Säugetiergewebe, dadurch charakterisiert, dass der Marker eine Kombination aus einer Mehrzahl von γ-Glu-X Peptiden (X steht für eine Aminosäure) ist.

4. Die Verwendung eines Markers für Lebererkrankungen gemäß Anspruch 3, dadurch charakterisiert, dass der Marker der Marker für Lebererkrankungen zur Identifizierung einer Arzneimittel-induzierten Hepatitis ist, der eine Kombination ist, die wenigstens γ-Glu-Thr, γ-Glu-Leu, γ-Glu-His und γ-Glu-Phe enthält.

5. Die Verwendung eines Markers für Lebererkrankungen gemäß Anspruch 3, dadurch charakterisiert, dass der Marker der Marker für Lebererkrankungen zur Identifizierung von Leberkrebs ist, der eine Kombination ist, die wenigstens γ-Glu-Val, γ-Glu-Thr, γ-Glu-Leu, γ-Glu-Phe und γ-Glu-Tyr enthält.

6. Die Verwendung eines Markers für Lebererkrankungen gemäß Anspruch 3, dadurch charakterisiert, dass der Marker der Marker für Lebererkrankungen zur Identifizierung eines asymptomatischen Hepatitis B Trägers ist, der ein Kombination ist, die wenigstens γ-Glu-Val, γ-Glu-Gln, γ-Glu-His und γ-Glu-Phe enthält.

7. Die Verwendung eines Markers für Lebererkrankungen gemäß Anspruch 1, dadurch charakterisiert, dass der Marker der Marker für Lebererkrankungen zur Identifizierung chronischer Hepatitis B ist, der wenigstens γ-Glu-Lys enthält.

**8.** Die Verwendung eines Markers für Lebererkrankungen gemäß Anspruch 3, dadurch charakterisiert, dass der Marker der Marker für Lebererkrankungen zur Identifizierung eines asymptomatischen Hepatitis C Trägers ist, der eine Kombination ist, die wenigstens AST, γ-Glu-Gly und γ-Glu-Phe enthält.

**9.** Die Verwendung eines Markers für Lebererkrankungen gemäß Anspruch 1, dadurch charakterisiert, dass der Marker der Marker für Lebererkrankungen zur Identifizierung chronischer Hepatitis C ist, der eine Kombination ist, die wenigstens γ-Glu-Ser, γ-Glu-Phe und γ-Glu-Tyr enthält.

**10.** *In vitro* Verwendung eines Markers für Lebererkrankungen zur Unterscheidung zwischen einer nicht-alkoholischen Fetterlebererkrankung (NAFLD), nicht-alkoholischen Steatohepatitis (NASH) und einfacher Steatose (SS), dadurch charakterisiert, dass der Marker ausgewählt wird aus der Gruppe γ-Glu-Gly, γ-Glu-Ala, γ-Glu-Ser, γ-Glu-Val, γ-Glu-Thr, γ-Glu-Taurin, γ-Glu-Ile, γ-Glu-Asn, γ-Glu-Asp, γ-Glu-Gln, γ-Glu-Lys, γ-Glu-Glu, γ-Glu-Met, γ-Glu-His, γ-Glu-Trp, γ-Glu-Arg und γ-Glu-Citrullin.

**11.** Ein *in vitro* Verfahren zum Nachweis von oxidativem Stress, dadurch charakterisiert, dass wenigstens eines von γ-Glu-Gly, γ-Glu-Ala, γ-Glu-Ser, γ-Glu-Val, γ-Glu-Thr, γ-Glu-Taurin, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrullin und γ-Glu-Tyr in einer Probe gemessen wird.

**12.** Ein Verfahren zur Untersuchung eines pharmazeutischen Erzeugnisses, dadurch charakterisiert, dass es die folgenden Schritte umfasst:

Messung einer Konzentration des Biomarkers für Lebererkrankungen, wie in irgendeinem der Ansprüche 1 bis 11 beschrieben, in humanem Blut, das vor und nach der Anwendung des pharmazeutischen Erzeugnisses erhalten wurde; und
Vergleichen der Messresultate zwischen dem Blut vor der Anwendung des oben genannten pharmazeutischen Erzeugnisses und dem Blut nach der Anwendung davon.

**13.** Ein Verfahren zur Untersuchung eines pharmazeutischen Erzeugnisses, dadurch charakterisiert, dass es die folgenden Schritte umfasst:

Messung einer Konzentration eines Markers einer Lebererkrankung, wie in irgendeinem der Ansprüche 1 bis 11 beschrieben, in Blut, das von einer ersten Gruppe erhalten wurde, die aus einem oder mehreren Individuen besteht, die das pharmazeutische Erzeugnis erhalten haben und Blut, das von einer zweiten Gruppe erhalten wurde, die aus einem oder mehreren Individuen besteht, die nicht das pharmazeutische Erzeugnis erhalten haben; und
Vergleichen der Konzentrationen der gemessenen Marker für Lebererkrankungen zwischen der ersten Gruppe und der zweiten Gruppe.

**14.** Ein Verfahren zum Nachweis von oxidativem Stress, dadurch charakterisiert, dass eines von γ-Glu-Gly, γ-Glu-Ala, γ-Glu-Ser, γ-Glu-Val, γ-Glu-Thr, γ-Glu-Taurin, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrullin und γ-Glu-Tyr in einem Leberorgan, das von einem nicht-humanen Säuger erhalten wurde, gemessen wird.

**15.** Ein Verfahren zum Nachweis von Hepatitis B oder C, wobei das Verfahren die folgenden Schritte umfasst:

Erhalten von Blut von einem oder mehreren Menschen;
Messen der Konzentration von γ-Glu-X in dem erhaltenen Blut; und
Vergleichen der Konzentration des Markers mit der im Blut von einem oder mehreren normalen Menschen.

**16.** Das Verfahren zum Nachweis von Hepatitis B der C gemäß Anspruch 15, worin die Hepatitis eine asymptomatische Hepatitis oder eine chronische Hepatitis ist.

**17.** Das Verfahren zum Nachweis von Hepatitis B oder C gemäß Ansprüchen 15 oder 16, wobei das γ-Glu-X ausgewählt wird aus der Gruppe bestehend aus γ-Glu-Gly, γ-Glu-Ser, γ-Glu-Val, γ-Glu-Thr, γ-Glu-Taurin, γ-Glu-Leu, γ-Glu-Gln, γ-Glu-Lys, γ-Glu-Glu, γ-Glu-His, γ-Glu-Phe und γ-Glu-Tyr.

## Revendications

1. Utilisation *in vitro* d'au moins un marqueur de maladie hépatique sélectionné dans l'ensemble constitué par γ-Glu-Gly, γ-Glu-Ala, γ-Glu-Ser, γ-Glu-Val, γ-Glu-Thr, γ-Glu-Taurine, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrulline et γ-Glu-Tyr pour détecter un stress oxydant dans un tissu de mammifère.

2. Utilisation d'au moins un marqueur de maladie hépatique selon la revendication 1, **caractérisée en ce que** le marqueur est le marqueur de maladie hépatique pour identifier une personne normale, qui est sélectionné dans l'ensemble constitué par γ-Glu-Phe, γ-Glu-Ser, γ-Glu-Thr, γ-Glu-Gly et γ-Glu-Glu.

3. Utilisation *in vitro* d'un marqueur de maladie hépatique pour détecter un stress oxydant dans un tissu de mammifère, **caractérisée en ce que** le marqueur est une combinaison d'une pluralité de peptides de type γ-Glu-X (où X représente un acide aminé).

4. Utilisation d'un marqueur de maladie hépatique selon la revendication 3, **caractérisée en ce que** le marqueur est le marqueur de maladie hépatique pour identifier une hépatite d'origine médicamenteuse, qui est une combinaison comprenant au moins γ-Glu-Thr, γ-Glu-Leu, γ-Glu-His et γ-Glu-Phe.

5. Utilisation d'un marqueur de maladie hépatique selon la revendication 3, **caractérisée en ce que** le marqueur est le marqueur de maladie hépatique pour identifier un cancer du foie, qui est une combinaison comprenant au moins γ-Glu-Val, γ-Glu-Thr, γ-Glu-Leu, γ-Glu-Phe et γ-Glu-Tyr.

6. Utilisation d'un marqueur de maladie hépatique selon la revendication 3, **caractérisée en ce que** le marqueur est le marqueur de maladie hépatique pour identifier un porteur asymptomatique de l'hépatite B, qui est une combinaison comprenant au moins γ-Glu-Val, γ-Glu-Gln, γ-Glu-His et γ-Glu-Phe.

7. Utilisation d'un marqueur de maladie hépatique selon la revendication 1, **caractérisée en ce que** le marqueur est le marqueur de maladie hépatique pour identifier une hépatite B chronique, qui comprend au moins γ-Glu-Lys.

8. Utilisation d'un marqueur de maladie hépatique selon la revendication 3, **caractérisée en ce que** le marqueur est le marqueur de maladie hépatique pour identifier un porteur asymptomatique de l'hépatite C, qui est une combinaison comprenant au moins AST, γ-Glu-Gly et γ-Glu-Phe.

9. Utilisation d'un marqueur de maladie hépatique selon la revendication 1, **caractérisée en ce que** le marqueur est le marqueur de maladie hépatique pour identifier une hépatite C chronique, qui est une combinaison comprenant au moins γ-Glu-Ser, γ-Glu-Phe et γ-Glu-Tyr.

10. Utilisation *in vitro* d'un marqueur de maladie hépatique pour établir une distinction entre une stéatose isolée, soit Non-Alcoholic Fatty Liver Disease (NAFLD), une stéatose non alcoolique, soit Non-Alcoholic SteatoHepatitis (NASH), et une stéatose simple, soit Simple Steatosis (SS), **caractérisée en ce que** le marqueur est sélectionné dans l'ensemble constitué par γ-Glu-Gly, γ-Glu-Ala, γ-Glu-Ser, γ-Glu-Val, γ-Glu-Thr, γ-Glu-Taurine, γ-Glu-Ile, γ-Glu-Asn, γ-Glu-Asp, γ-Glu-Gln, γ-Glu-Lys, γ-Glu-Glu, γ-Glu-Met, γ-Glu-His, γ-Glu-Trp, γ-Glu-Arg et γ-Glu-Citrulline.

11. Procédé *in vitro* de détection d'un stress oxydant, **caractérisé en ce qu'**au moins un composé parmi γ-Glu-Gly, γ-Glu-Ala, γ-Glu-Ser, γ-Glu-Val, γ-Glu-Thr, γ-Glu-Taurine, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrulline et γ-Glu-Tyr est mesuré dans un échantillon.

12. Procédé d'analyse d'une préparation pharmaceutique, **caractérisé en ce qu'**il comprend les étapes suivantes :

    mesure d'une concentration du biomarqueur de maladie hépatique selon l'une quelconque des revendications 1 à 11 dans du sang humain collecté avant et après l'administration de la préparation pharmaceutique ; et
    comparaison des résultats de mesure entre le sang avant l'administration de la préparation pharmaceutique susmentionnée et le sang après l'administration de celle-ci.

13. Procédé d'analyse d'une préparation pharmaceutique, **caractérisé en ce qu'**il comprend les étapes suivantes :

mesure d'une concentration du marqueur de maladie hépatique selon l'une quelconque des revendications 1 à 11 dans du sang collecté parmi un premier groupe constitué par un ou plusieurs individus qui ont reçu la préparation pharmaceutique, et du sang collecté parmi un deuxième groupe constitué par un ou plusieurs individus qui n'ont pas reçu la préparation pharmaceutique ; et
comparaison des concentrations du marqueur de maladie hépatique mesurées entre le premier groupe et le deuxième groupe.

**14.** Procédé de détection d'un stress oxydant, **caractérisé par** la mesure d'un composé parmi γ-Glu-Gly, γ-Glu-Ala, γ-Glu-Ser, γ-Glu-Val, γ-Glu-Thr, γ-Glu-Taurine, γ-Glu-Ile, γ-Glu-Leu, γ-Glu-Asn, γ-Glu-Asp, γ-Glu-Gln, γ-Glu-Lys, γ-Glu-Glu, γ-Glu-Met, γ-Glu-His, γ-Glu-Phe, γ-Glu-Trp, γ-Glu-Arg, γ-Glu-Citrulline et γ-Glu-Tyr dans un organe de foie prélevé sur un mammifère non humain.

**15.** Procédé de détection de l'hépatite B ou C, le procédé comprenant les étapes suivantes :

collecte de sang sur un ou plusieurs individus humains ;
mesure de la concentration de γ-Glu-X dans le sang collecté ; et
comparaison de la concentration du marqueur à celle présente dans le sang d'un ou plusieurs individus normaux.

**16.** Procédé de détection de l'hépatite B ou C selon la revendication 15, dans lequel l'hépatite est une hépatite asymptomatique ou une hépatite chronique.

**17.** Procédé de détection de l'hépatite B ou C selon la revendication 15 ou 16, dans lequel le composé γ-Glu-X est sélectionné dans l'ensemble constitué par γ-Glu-Gly, γ-Glu-Ser, γ-Glu-Val, γ-Glu-Thr, γ-Glu-Taurine, γ-Glu-Leu, γ-Glu-Gln, γ-Glu-Lys, γ-Glu-Glu, γ-Glu-His, γ-Glu-Phe et γ-Glu-Tyr.

Fig. 1

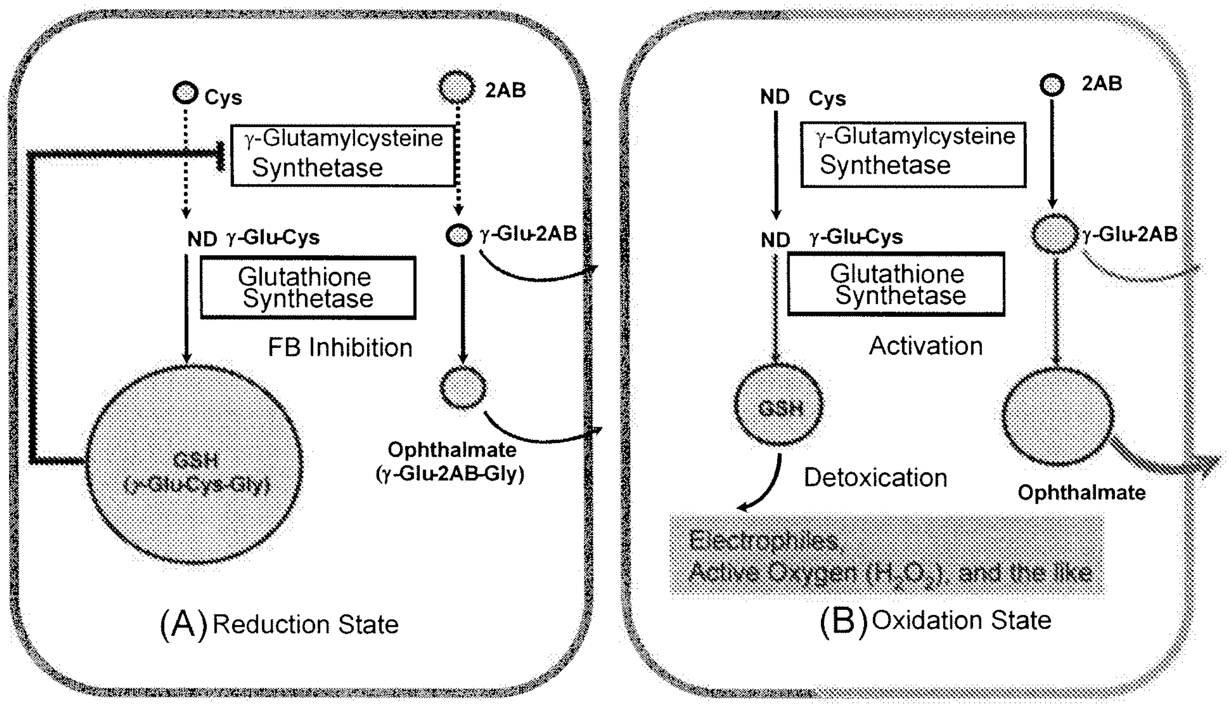
Cys
2AB
γ-Glutamylcysteine Synthetase
ND γ-Glu-Cys
γ-Glu-2AB
Glutathione Synthetase
FB Inhibition
GSH (γ-Glu-Cys-Gly)
Ophthalmate (γ-Glu-2AB-Gly)
(A) Reduction State
ND Cys
2AB
γ-Glutamylcysteine Synthetase
ND γ-Glu-Cys
γ-Glu-2AB
Glutathione Synthetase
Activation
GSH
Detoxication
Ophthalmate
Electrophiles
Active Oxygen ($H_2O_2$), and the like
(B) Oxidation State

Fig. 2

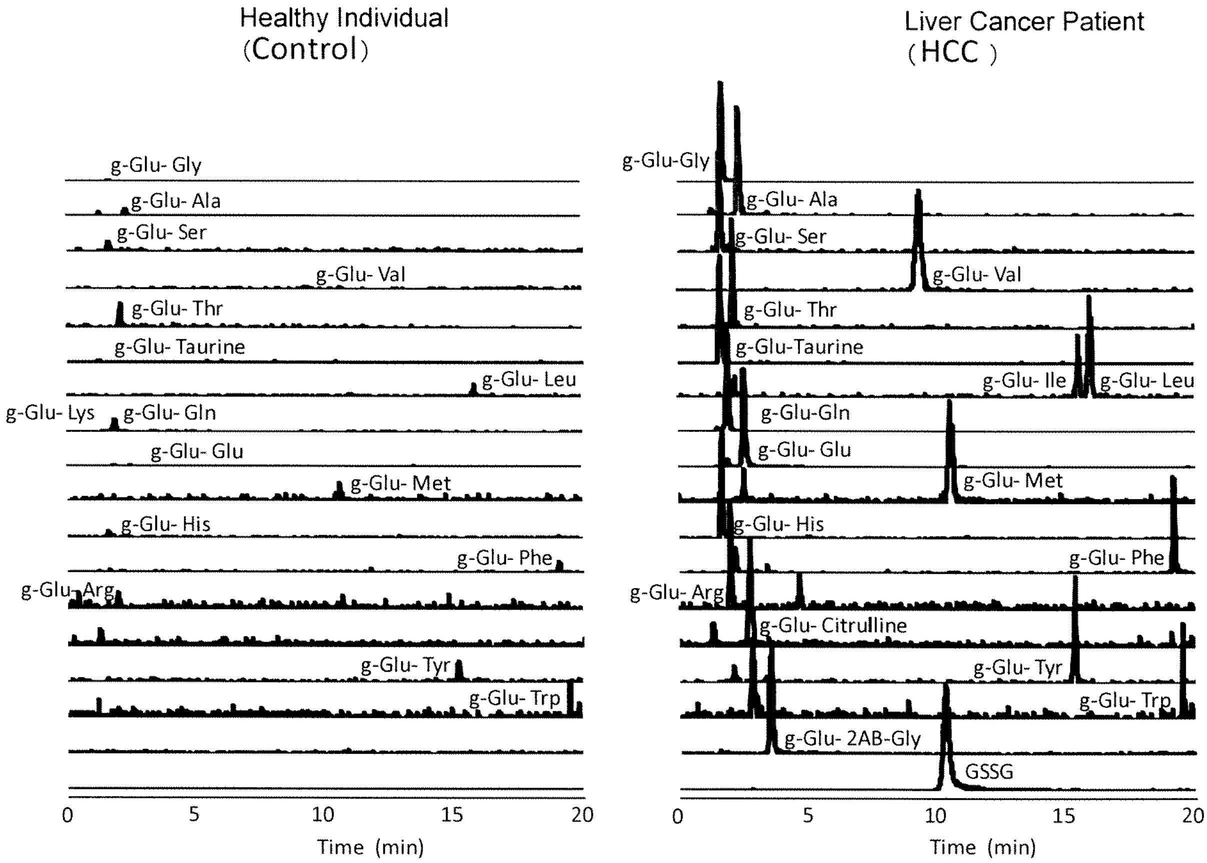
Healthy Individual
(Control)
g-Glu- Gly
g-Glu- Ala
g-Glu- Ser
g-Glu- Val
g-Glu- Thr
g-Glu- Taurine
g-Glu- Leu
g-Glu- Lys
g-Glu- Gln
g-Glu- Glu
g-Glu- Met
g-Glu- His
g-Glu- Phe
g-Glu- Arg
g-Glu- Tyr
g-Glu- Trp
0
5
10
15
20
Time (min)
Liver Cancer Patient
(HCC)
g-Glu-Gly
g-Glu- Ala
g-Glu- Ser
g-Glu- Val
g-Glu- Thr
g-Glu-Taurine
g-Glu- Ile
g-Glu- Leu
g-Glu-Gln
g-Glu- Glu
g-Glu- Met
g-Glu- His
g-Glu- Phe
g-Glu- Arg
g-Glu- Citrulline
g-Glu- Tyr
g-Glu- Trp
g-Glu- 2AB-Gly
GSSG
0
5
10
15
20
Time (min)

Fig. 3

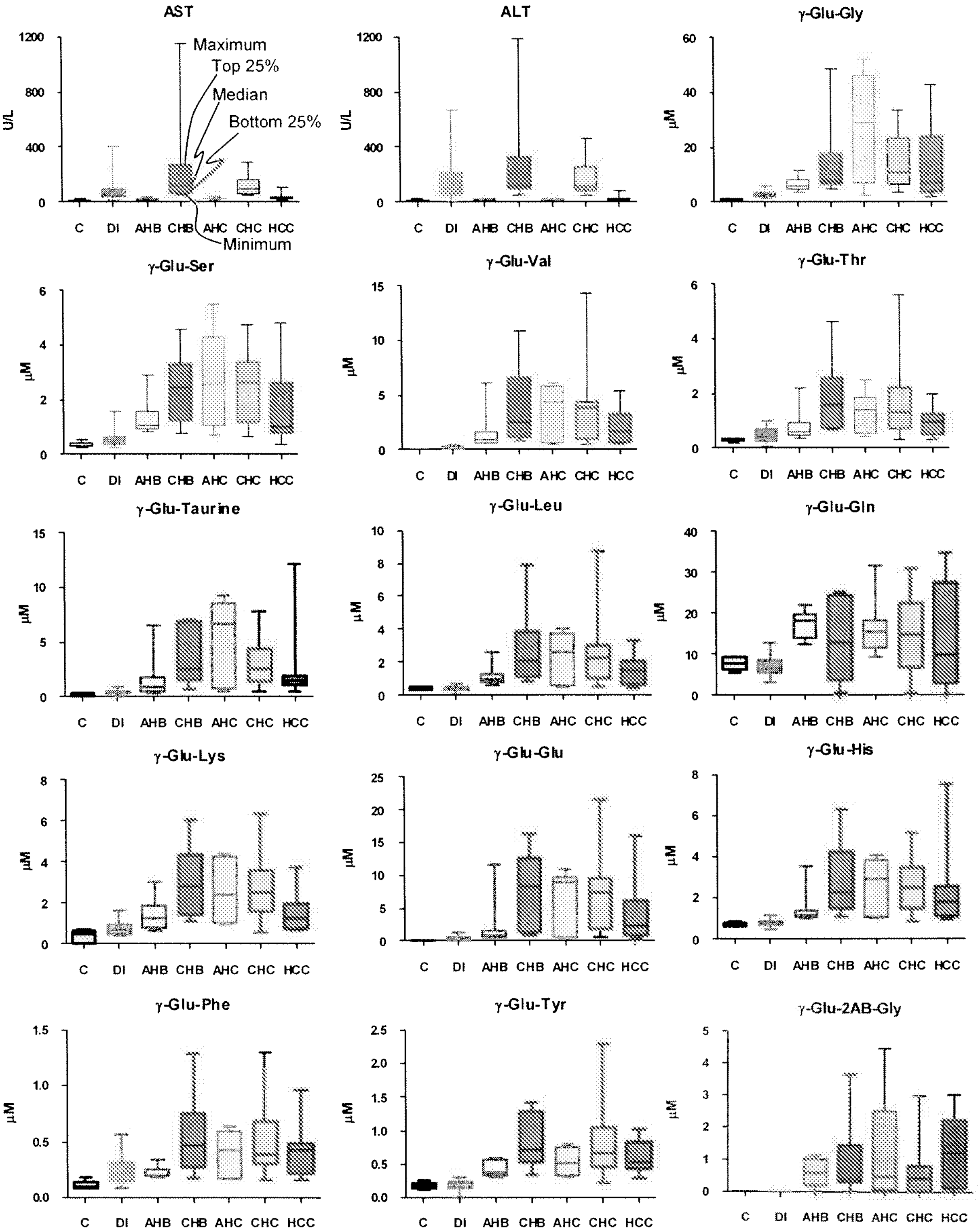
AST
ALT
γ-Glu-Gly
Maximum
Top 25%
Median
Bottom 25%
Minimum
U/L
μM
C DI AHB CHB AHC CHC HCC
γ-Glu-Ser
γ-Glu-Val
γ-Glu-Thr
γ-Glu-Taurine
γ-Glu-Leu
γ-Glu-Gln
γ-Glu-Lys
γ-Glu-Glu
γ-Glu-His
γ-Glu-Phe
γ-Glu-Tyr
γ-Glu-2AB-Gly

Fig. 4

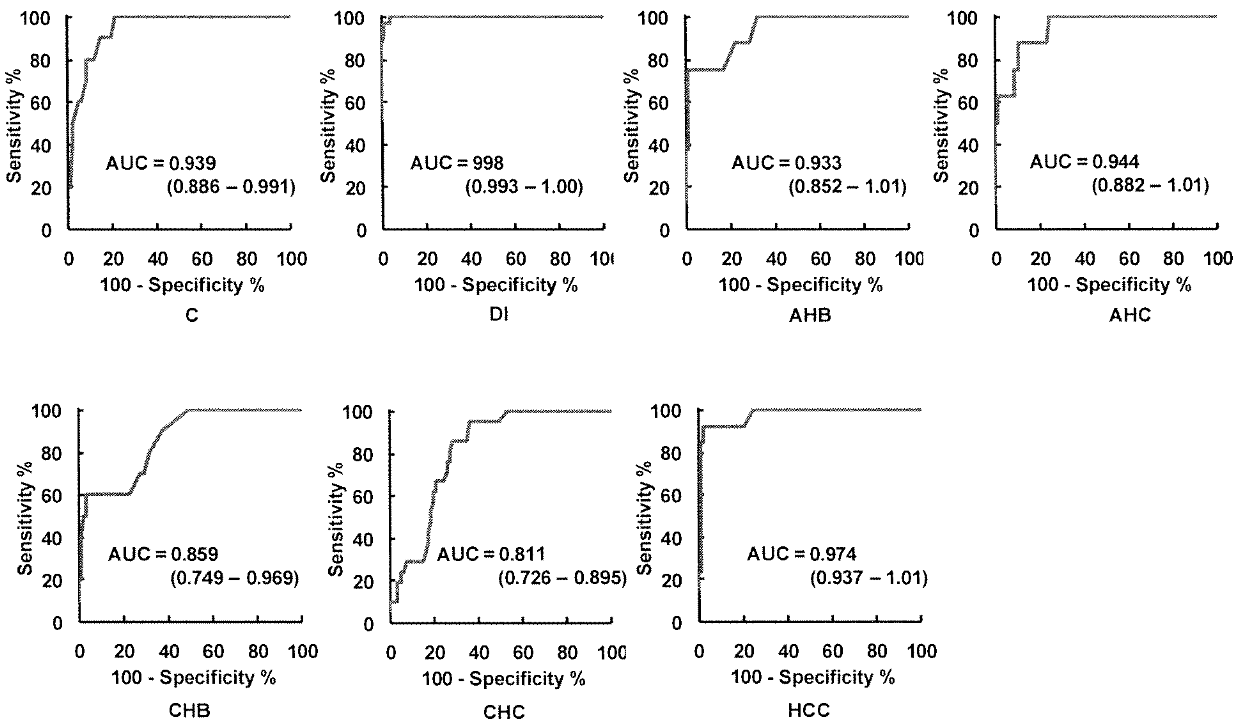
Sensitivity %
100 - Specificity %
AUC = 0.939
(0.886 – 0.991)
C
AUC = 998
(0.993 – 1.00)
DI
AUC = 0.933
(0.852 – 1.01)
AHB
AUC = 0.944
(0.882 – 1.01)
AHC
AUC = 0.859
(0.749 – 0.969)
CHB
AUC = 0.811
(0.726 – 0.895)
CHC
AUC = 0.974
(0.937 – 1.01)
HCC

Fig. 5

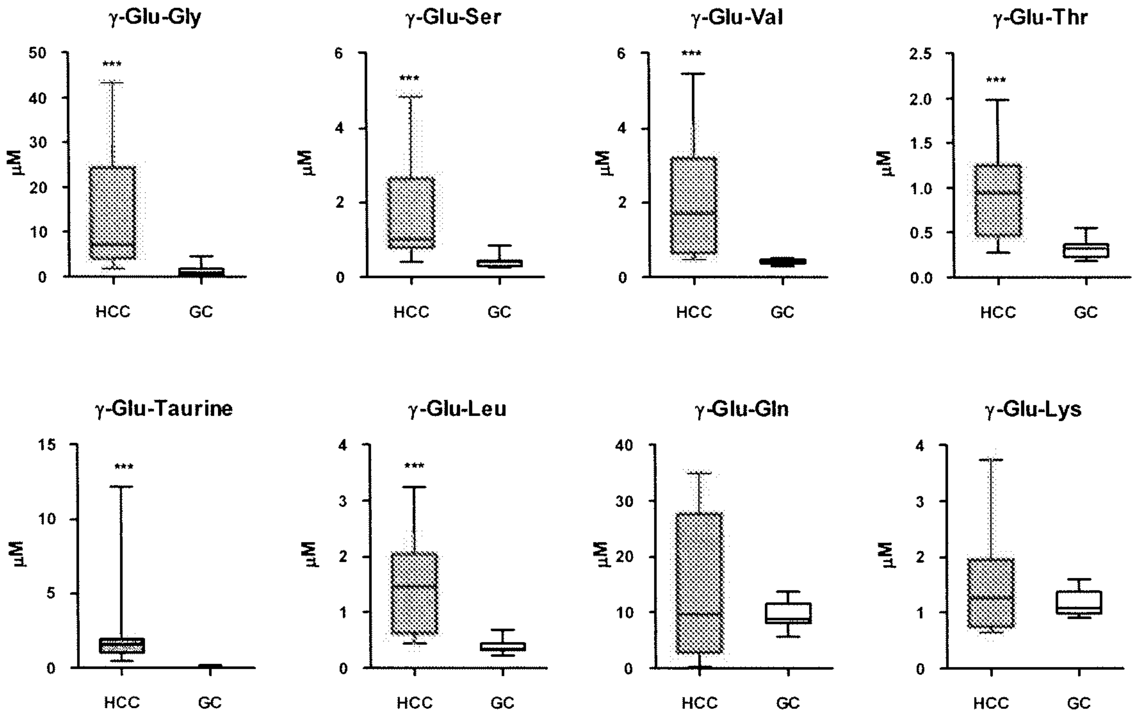
γ-Glu-Gly
γ-Glu-Ser
γ-Glu-Val
γ-Glu-Thr
γ-Glu-Taurine
γ-Glu-Leu
γ-Glu-Gln
γ-Glu-Lys
μM
HCC
GC
***

Fig. 6

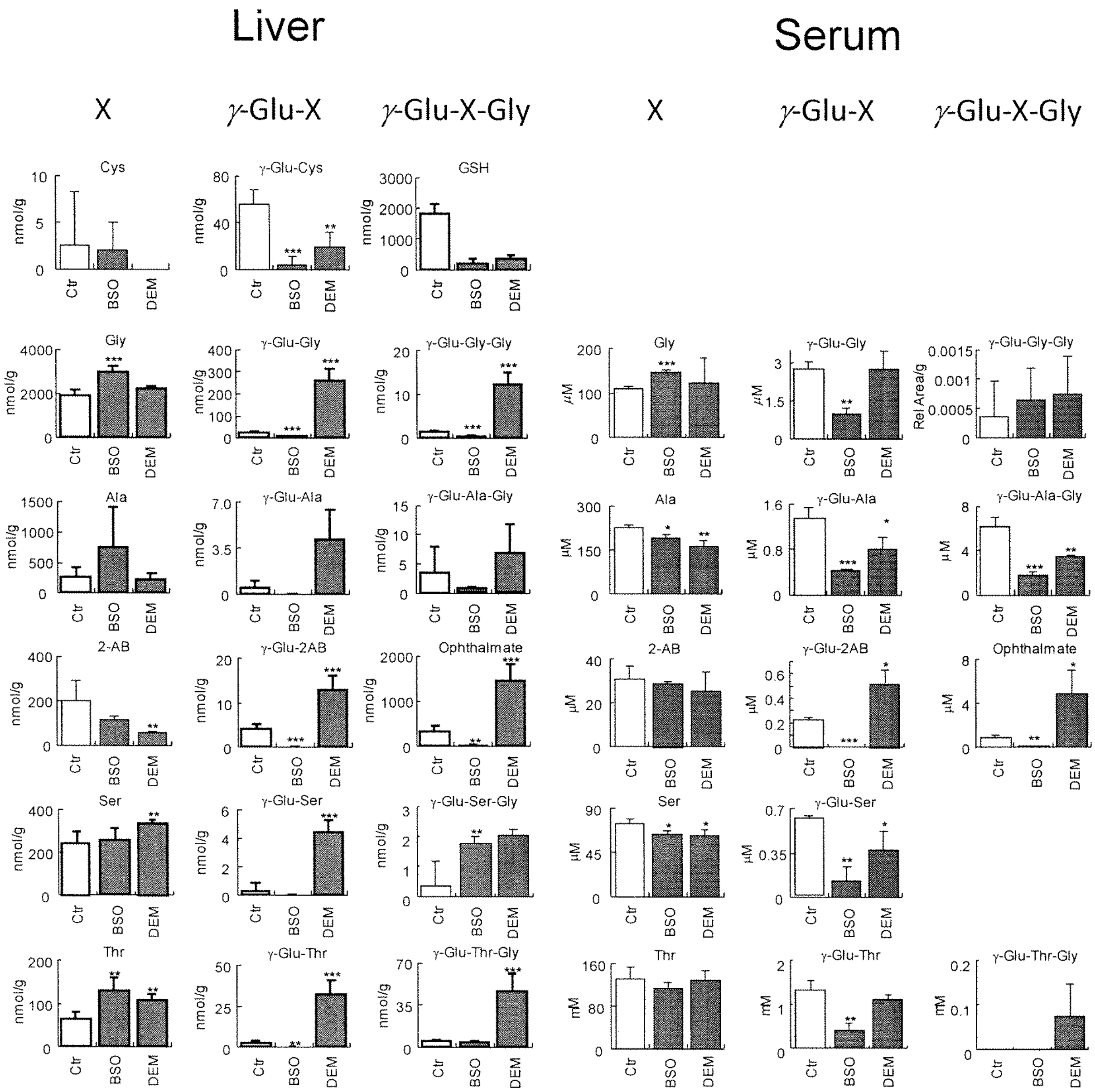
Liver
Serum
X
γ-Glu-X
γ-Glu-X-Gly
Cys
γ-Glu-Cys
GSH
Gly
γ-Glu-Gly
γ-Glu-Gly-Gly
Ala
γ-Glu-Ala
γ-Glu-Ala-Gly
2-AB
γ-Glu-2AB
Ophthalmate
Ser
γ-Glu-Ser
γ-Glu-Ser-Gly
Thr
γ-Glu-Thr
γ-Glu-Thr-Gly
nmol/g
μM
mM
Rel Area/g
Ctr
BSO
DEM

Fig. 7

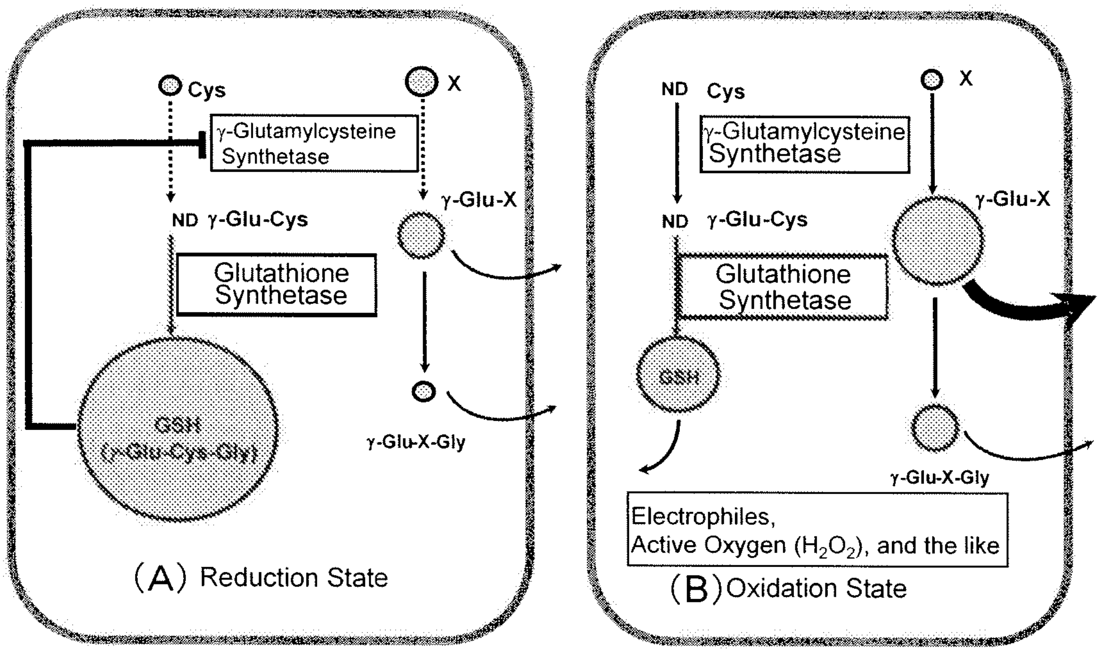
Cys
X
γ-Glutamylcysteine Synthetase
ND γ-Glu-Cys
γ-Glu-X
Glutathione Synthetase
GSH (γ-Glu-Cys-Gly)
γ-Glu-X-Gly
(A) Reduction State
ND Cys
X
γ-Glutamylcysteine Synthetase
ND γ-Glu-Cys
γ-Glu-X
Glutathione Synthetase
GSH
γ-Glu-X-Gly
Electrophiles,
Active Oxygen ($H_2O_2$), and the like
(B) Oxidation State

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007192746 A
- JP 3341765 B

### Non-patent literature cited in the description


- **CALLEWAERT, N. et al.** *Nat. Med.,* 2004, vol. 10, 429-434
- **LOGUERCIO, CARMELA et al.** *Free Radic. Biol. Med.,* 2003, vol. 34, 1-10
- **YADAV, DHIRAJ et al.** *Am. J. Gastroenterol.,* 2002, vol. 97, 2634-2639
- **MORIYA, K. et al.** *Cancer Res.,* 2001, vol. 61, 4365-4370
- **SOGA, T. et al.** *J. Biol. Chem.,* 2006, vol. 281, 16768-16776
- Sanka Storesu to Kanshikkan. Oxidative Stress and Liver Diseases. Medical Tribune, Inc, 07 May 2009, vol. 5, 3-37
- **SOGA, T. et al.** *J. Proteome Res.,* 2003, vol. 2, 488-494
- **SOGA, T. et al.** *J. Boil Chem.,* 16 June 2006, vol. 281 (24), 16768-16776
- **HIRAYAMA A. et al.** *Cancer. Res.,* 01 June 2009, vol. 69, 4918-4925
- **PIGNATELLI, B. et al.** *Am. J. Gastroenterol.,* 2001, vol. 96, 1758-1766